# FASCICULE DE BREVET EUROPEEN

(11) **EP 1 517 918 B1**
(45) Date de publication et mention de la délivrance du brevet: **18.03.2009**
(21) Numéro de dépôt: 03761668.7
(22) Date de dépôt: 30.06.2003
(51) Int. Cl.: C07K 14/47, A61M 1/34, A61M 1/36, C12N 15/12, G01N 3/58, A61K 51/08, A61P 7/02, A61P 29/00

(54) **PEPTIDES AYANT UNE AFFINITE POUR UN PHOSPHOLIPIDE ET UTILISATIONS**
PEPTIDE MIT AFFINITÄT FÜR EIN PHOSPHOLIPID UND DEREN VERWENDUNG
PEPTIDES HAVING AFFINITY FOR A PHOSPHOLIPID AND USES

(30) Priorité: 01.07.2002 FR 0208202
(43) Date de publication de la demande: 30.03.2005
(73) Titulaire: COMMISSARIAT A L'ENERGIE ATOMIQUE, 75752 Paris Cédex 15 (FR); Université Pierre et Marie Curie (Paris VI), 75252 Paris Cédex 05 (FR)
(72) Inventeur: SANSON, Alain, F-91940 GOMETZ LE CHATEL (FR); OCHSENBEIN, Françoise, F-91190 Gif sur Yvette (FR)
(74) Mandataire: Poulin, Gérard
(86) Numéro de dépôt international: PCT/FR2003/002025
(87) Numéro de publication internationale: WO 2004/003015

(56) Documents cités:
- EP-A- 0 293 567
- WO-A-00/10673
- WO-A-00/20453
- WO-A-92/19279
- MONTAVILLE PIERRE ET AL: "A new consensus sequence for phosphatidylserine recognition by annexins." THE JOURNAL OF BIOLOGICAL CHEMISTRY. UNITED STATES 5 JUL 2002, vol. 277, no. 27, 5 juillet 2002 (2002-07-05), pages 24684-24693, XP002268388 ISSN: 0021-9258
- SABLE E.A.: "Cloning and functional activity of a novel truncated form of annexin IV in mouse macrophages" BIOCHEMICAL AND BIOPHYSICAL RESEARCH COMMUNICATIONS, vol. 258, no. 1, 1999, pages 162-167, XP002239575 ORLANDO, FL US

## Description

### DOMAINE TECHNIQUE

La présente invention se rapporte à une famille de peptides ayant une affinité pour un phospholipide ainsi qu'à diverses utilisations de ce peptide, notamment dans le domaine pharmaceutique.

De manière générale, les peptides de la présente invention sont utiles pour la reconnaissance spécifique de molécules lipidiques. Ils sont utilisables pour l'ingénierie et la création de composés de reconnaissance et de séquestration de lipides notamment de lipides chargés négativement, tels que les phosphatidylsérines, les acides phosphatidiques et lyso-phosphatidiques, les phosphatidylglycérols, les cardiolipines et les sphyngosines-1-phosphates.

Les lipides précités jouent un rôle important notamment dans la signalisation cellulaire et peuvent être présents à la surface externe des membranes des cellules et/ou circuler dans le milieu sanguin à la suite d'événements pathologiques très divers.

Divers événements cellulaires aboutissent à l'apparition de lipides chargés négativement et notamment de phosphatidylsérines (PS) à la surface externe des cellules, ces événements peuvent résulter soit d'une altération fortuite ou pathologique de la cellule, soit d'un événement cellulaire programmé telle que la mort cellulaire ou apoptose. L'apparition de PS à la surface externe des cellules constitue donc un "message primaire" important témoignant de l'existence d'un dysfonctionnement. Dans le cas du processus de coagulation sanguine, le mécanisme est bien décrit : l'altération des cellules endothéliales des vaisseaux sanguins, soit pour des raisons accidentelles, soit pour des raisons pathologiques plus complexes, provoque l'apparition de ce message PS à la surface externe des cellules en contact avec le milieu sanguin. Ce message est immédiatement reconnu par certaines protéines circulantes qui déclenchent alors une cascade d'événements aboutissant au phénomène de coagulation sanguine bien connu.

L'invention tire profit de la propriété des peptides qu'elle fournit de se lier, en présence ou non de calcium, aux lipides et notamment à ceux chargés négativement, pour la mise au point de composés utilisables comme outils de recherche, de diagnostic et de thérapeutique dans le domaine de la reconnaissance des effecteurs lipidiques et de la détection de l'apoptose, des troubles de la coagulation sanguine, du choc septique et des pathologies inflammatoires aiguës en particulier.

Concernant la recherche et le diagnostic, les peptides de l'invention peuvent par exemple être couplés à des molécules de détection, par exemple à une molécule fluorescente, à l'un des partenaires du système avidine-biotine, à un radioélément à vie courte, à un composé paramagnétique, à des particules d'or ou de composés denses pour la microscopie électronique. Avec ces molécules de détection, il est possible par exemple de détecter des cellules apoptotiques ou de reconnaître des microdomaines membranaires chargés négativement.

Les peptides de la présente invention peuvent donc être utilisés pour une détection "*in vitro*" de pathologies impliquant l'apparition de charges négatives à la surface des cellules et la libération dans le sang de microvésicules.

Les peptides de la présente invention peuvent également être utilisés pour la détection in vivo et l'imagerie des foyers apoptotiques, de zones thrombotiques, et de manière générale de tout centre exposant des lipides chargés négativement, lorsque ces peptides sont couplés par exemple à un radioélément à durée de vie courte (les images scintigraphiques, acquises en tomographie d'émission monophotonique (SPECT pour « Single Photon Emission Computed Tomography ») ou en tomographie par émission de positons (PET pour « Positron Emission Tomography »)) ou à un composé de contraste quelconque tel qu'un complexe de gadolinium pour l'imagerie à résonance magnétique (IRM).

Concernant la thérapeutique, de manière générale, les peptides de la présente invention peuvent être utilisés seuls ou couplés à une molécule thérapeutique pour préparer un médicament. Un tel médicament peut par exemple être utilisé pour le ciblage de cette molécule vers des zones présentant des charges négatives telles que des tumeurs présentant des foyers de cellules apoptotiques ou des tumeurs inflammatoires.

Les peptides de la présente invention peuvent par exemple être couplés à des molécules à action thrombolytique pour préparer un médicament utilisable dans le traitement et la prophylaxie de la thrombose, ou pour préparer une molécule recouvrant tous les biomatériaux thrombogènes. Les peptides de la présente invention peuvent donc être utilisés pour le ciblage des molécules thrombolytiques au site du thrombus ou vers les zones thrombogènes.

Dans un autre exemple d'application de la présente invention, les peptides de l'invention peuvent être utilisés seuls ou couplés à une molécule anti-inflammatoire pour préparer un médicament, utilisable par exemple dans des pathologies aiguës comme l'asthme, la rectocolite hémorragique (RCH), la maladie de Crohn, le choc septique, les maladies du collagène et de l'arthrite.

D'autres applications apparaîtront encore à l'homme du métier à la lecture de la description qui suit.

### ETAT DE LA TECHNIQUE

Une famille de protéines, appelées annexines, ont été décrites dans l'art antérieur comme présentant un ancrage fonctionnel réversible à la membrane cellulaire, régulé par la concentration en calcium et la présence de phospholipides anioniques. Les annexines constituent une famille de protéines exprimées dans des tissus très divers, aussi bien chez les animaux que chez les plantes. Il semble qu'elles ne sont ni exprimées chez la bactérie, ni chez la levure.

La structure des annexines comporte quatre domaines d'environ 70 acides aminés, ou résidus, très moyennement homologues en séquence mais de topologie quasiment identique.

Dans le document WO 92/19279, J. TAIT décrit des conjugués ayant une affinité pour des phospholipides. Il décrit en particulier l'utilisation d'une annexine, en particulier de l'annexine V, pour fabriquer un conjugué actif utilisable en tant qu'agent thrombolytique.

Malheureusement, le composé décrit dans ce document et préparé à partir de l'annexine entière par un procédé de recombinaison génétique, possède de nombreux inconvénients qui sont notamment un rendement faible, un coût de fabrication élevé. Les inconvénients majeurs sont surtout l'obtention d'un conjugué fragile du fait de sa topologie complexe conduisant à un dépliement irréversible. En outre, ces molécules présentent une toxicité majeur pour le rein et le coeur.

L'article de Montaville et al. 2002 (J. Biol. Chem., vol. 277, pages 24684-24693) décrit une séquence consensus grandement conservée parmi les annexines et responsable de leur liaison aux membranes exposant des phospholipides.

Les présents inventeurs ont décrit dans la demande WO-A-00/20453 une première famille de peptides palliant les inconvénients précités et présentant une affinité pour les phospholipides et une stabilité améliorées.

### EXPOSE DE L'INVENTION

La présente invention.a pour but de fournir une nouvelle famille de peptides ayant une affinité pour les lipides, en particulier pour les phospholipides, plus spécifique et encore améliorée par rapport aux produits de l'art antérieur.

Les peptides de l'invention présentent en outre les avantages d'être plus stables chimiquement que les composés de l'art antérieur et de pouvoir être fabriqués de manière reproductible, avec un rendement élevé et un coût de fabrication très réduit par rapport aux composés de l'art antérieur.

Les peptides de la présente invention sont tels que définis à l'une quelconque des revendications 1 à 8.

L'invention décrit également des peptides comprenant la séquence peptidique (I) suivante :

J¹-J²-J³-J⁴-J⁵-J⁶-J⁷-J⁸-J⁹-J¹⁰-J¹¹-Arg-J¹³-J¹⁴-U¹⁵-Lys-Gly-X¹⁸-Gly-Thr-J²¹-Glu-J²³-J²⁴-U²⁵-J²⁶-J²⁷-J²⁸-U²⁹-J³⁰-J³¹-Arg-J³³-J³⁴-J³⁵-J³⁶-B³⁷-J³⁸-J³⁹-U⁴⁰-J⁴¹-J⁴²-J⁴³-U⁴⁴-J⁴⁵-J⁴⁶-J⁴⁷-J⁴⁸-J⁴⁹-Arg-J⁵¹-U⁵²-J⁵³-J⁵⁴-Asp-U⁵⁶-Lys-Ser-Z⁵⁶-Leu-J⁶¹-J⁶²-J⁶³-J⁶⁴-Z⁶⁵-J⁶⁶-J⁶⁷-U⁶⁸-J⁶⁹-J⁷⁰-J⁷¹-U⁷²-J⁷³-J⁷⁵ (I)

dans laquelle J, Z, U, X et B représentent des acides aminés tels que :
- les acides aminés J sont choisis indépendamment les uns des autres parmi les acides aminés naturels, ou des dérivés de ceux-ci, de telle manière qu'au moins 50% d'entre eux sont des résidus polaires choisis parmi Arg, Asn, Asp, Cys, Gln, Glu, Gly, His, Lys, Orn, Pro, Ser, Thr et Tyr,
- les acides aminés U sont choisis parmi Ala, Cys, Gly, Ile, Leu, Met, Phe, Trp, Tyr et Val,
- l'acide aminé X¹⁸ est choisi indépendamment des autres acides aminés de la séquence parmi Ala, Asn, Cys, Gln, Gly, His, Ile, Leu, Met, Phe, Ser, Thr, Trp, Tyr et Val,
- l'acide aminé B³⁷ est choisi indépendamment des autres acides aminés de la séquence parmi Arg, Ala, Cys, Gly, Ile, Leu, Met, Phe, Trp, Tyr et Val,
- l'acide aminé Z⁷ est choisi indépendamment des autres acides aminés de la séquence parmi Asp et Glu,
- les acides aminés Z⁵⁹ et Z⁶⁵ sont indépendamment Glu, Asp, Lys ou Arg,
les exposants des J, Z, U, X et B représentant la position de ces acides aminés dans ladite séquence.

La séquence peptidique ci-dessus se replie dans l'espace pour adopter sa conformation tertiaire qui est la forme active du peptide, tout comme la séquence des peptides de l'invention.

Les acides aminés 12, 15, 16, 17, 19, 20, 22, 50, 55, 57, 58, 59, 60 et 65 sont les acides aminés, ou résidus, de la présente invention impliqués directement ou indirectement dans la liaison aux lipides, c'est-à-dire qu'ils sont impliqués soit dans la structure tridimensionnelle du peptide pour qu'il adopte sa conformation active permettant de reconnaître un lipide chargé négativement, soit dans le site de reconnaissance du peptide.

Les acides aminés J sont les acides aminés, ou résidus, de surface de ce peptide lorsqu'il est dans sa conformation repliée et active. Ces résidus sont disposés dans l'espace de telle sorte qu'ils sont partiellement ou totalement exposés au solvant. Selon la présente invention, ces acides aminés J peuvent être par exemple choisis indépendamment les uns des autres parmi l'ensemble des résidus aminoacides naturels Ala, Arg, Asn, Asp, Cys, Gln, Glu, Gly, His, Ile, Leu, Lys, Met, Orn, Phe, Pro, Ser, Thr Trp, Tyr et Val, et de telle manière que au moins 50% d'entre eux sont des résidus polaires choisis parmi Arg, Asn, Asp, Cys, Gln, Glu, Gly, His, Lys, Orn, Pro, Ser et Thr. Des exemples sont donnés dans la liste de séquences annexée.

Les acides aminés U sont les résidus de coeur de ce peptide. Dans la conformation repliée et active du peptide, ils sont disposés dans l'espace proches les uns des autres et non exposés au solvant. Ils constituent le coeur hydrophobe de la protéine. L'assemblage compact des atomes de ces résidus joue un rôle prédominant pour la stabilité du peptide dans sa conformation active. Ces résidus peuvent être choisis dans la liste d'acides aminés U décrite ci-dessus. Différents exemples de combinaisons de résidus de coeur dans le peptide de séquence (I) sont donnés dans le tableau (1) ci-dessous:

**Tableau 1**

| | **U⁸** | **U¹¹** | **U¹⁵** | **U²⁵** | **U²⁹** | **B³⁷** | **U⁴⁰** | **U⁴⁴** | **U⁵²** | **U⁵⁶** | **U⁶⁸** | **U⁷²** |
|---|---|---|---|---|---|---|---|---|---|---|---|---|
| **Ex a)** | Val | Leu | Met | Ile | Leu | Arg | Ile | Tyr | Leu | Leu | Val | Leu |
| **Ex b)** | Ala | Ile | Ile | Ile | Leu | Arg | Ile | Tyr | Leu | Leu | Ile | Leu |
| **Ex c)** | Ala | Ile | Ile | Ile | Leu | Arg | Ile | Tyr | Leu | Leu | Met | Val |
| **Ex d)** | Ala | Leu | Met | Leu | Leu | Arg | Ile | Tyr | Leu | Leu | Ile | Met |
| **Ex e)** | Ala | Leu | Met | Ile | Ile | Arg | Val | Tyr | Leu | Leu | Ile | Met |
| **Ex f)** | Ala | Leu | Met | Ile | Ile | Arg | Ile | Phe | Leu | Leu | Ile | Met |
| **Ex g)** | Ala | Leu | Met | Ile | Val | Arg | Ile | Phe | Leu | Leu | Ile | Phe |
| **Ex h)** | Val | Leu | Met | Ile | Leu | Arg | Ile | Phe | Leu | Leu | Ile | Met |
| **Ex i)** | Ala | Leu | Met | Ile | Leu | Arg | Ile | Phe | Leu | Leu | Ile | Met |
| **Ex j)** | Ala | Leu | Met | Ile | Leu | Arg | Ile | Tyr | Leu | Leu | Ala | Ala |
| **Ex k)** | Val | Leu | Met | Ile | Leu | Arg | Ile | Tyr | Leu | Leu | Val | Leu |
| **Ex l)** | Val | Leu | Met | Ile | Leu | Arg | Ile | Phe | Leu | Leu | Val | Leu |

| | | | | | | | | | | | | |
|---|---|---|---|---|---|---|---|---|---|---|---|---|
| (Ex = exemple) | | | | | | | | | | | | |

Le résidu X¹⁸ a pour fonction de maintenir la structure de la boucle Gly-X-Gly dans la forme active du peptide, notamment où les résidus Z⁵⁹ et Z⁶⁵ sont des Glu, de moduler le caractère hydrophobe et lipophile de cette boucle, et d'assurer éventuellement des interactions nouvelles spécifiques avec les phospholipides. Ceci est le cas par exemple des résidus Asn, Cys, Ser, Thr, Trp et Tyr.

Les résidus Z⁵⁹ et Z⁶⁵ peuvent être avantageusement des résidus lysine, ce qui a pour effet de remplacer l'ion calcium par le groupe chargé positivement -NH₃⁺ de la lysine et d'améliorer l'affinité du peptide pour une membrane chargée négativement.

Comme le peptide (I), le peptide de la présente invention, dans sa forme active comprend trois sites de liaison à un ion calcium où l'ion calcium complexé par ce site constitue un des ligands d'un phospholipide chargé négativement. Le premier de ces sites, appelé site principal, fait intervenir les résidus 15, 18, 19 et 59 en tant que ligands du calcium. Le deuxième dé ces sites, appelé site secondaire, fait intervenir les résidus 20 et 22 en tant que ligands du calcium. Le troisième de ces sites, qui est un site secondaire de, faible affinité, fait intervenir les résidus 57, 60, et 65 en tant que ligands du calcium.

Les résidus globalement impliqués dans la liaison aux phospholipides sont les résidus 12, 15, 16, 19, 20, 22, 50, 55, 57, 58, 59, 60 et 65. Cette liste inclut des résidus impliqués dans les liaisons du calcium, les phospholipides étant des ligands du calcium.

Ces résidus peuvent bien entendu être remplacés par des résidus remplissant la même fonction en vue du même résultat conformément à la présente invention.

A titre d'exemple, selon l'invention, le peptide de formule (I) peut être avantageusement une séquence peptidique choisie parmi les séquences peptidique ID n°1 à ID n°10 annexées.

La séquence de la revendication 1 représente les peptides de la présente invention dans leur forme fonctionnelle la plus courte. Il est bien entendu que cette séquence peut comprendre en outre, liée à l'extrémité N-terminale et/ou à l'extrémité C-terminale de la séquence, un ou plusieurs acides aminés, par exemple de 1 à 15 acides aminés, en général de 1 à 10 acides aminés. De toute préférence, ces acides aminés complémentaires ne modifient pas ou peu l'activité des peptides, ou.alors l'améliorent.

Par exemple, une petite séquence, appelée ci-dessous séquence de fonctionnalisation peut être utile notamment pour fixer un marqueur sur le peptide, pour fixer une molécule de traitement de maladies sur le peptide et/ou pour fixer ledit peptide sur un support. La longueur de cette séquence de fonctionnalisation sera adaptée suivant son usage. Bien entendu, celle-ci n'interfèrera de préférence pas avec l'activité du peptide de la présente invention. L'homme du métier saura facilement adapter la longueur et la nature de cette séquence de fonctionnalisation suivant l'utilisation qu'il fera d'un peptide de la présent invention.

Ainsi, selon un premier mode particulier de réalisation de la présente invention, les peptides de la présente invention peuvent comporter, par exemple à leur extrémité N-terminale, une séquence de fonctionnalisation de trois acides aminés. Cette séquence de fonctionnalisation permet une fixation directe d'une molécule de traitement de maladies sur le peptide et/ou la fixation directe dudit peptide sur un support. Les peptides conformes à ce mode de réalisation peuvent être définis par la séquence (II) suivante :

J⁻²-J⁻¹-J⁰-J¹-J²-J³-J⁴-J⁵-J⁶-Asp-U⁸-J⁹-J¹⁰-U¹¹-Arg-J¹³-Ala-U¹⁵-Lys-Gly-X¹⁸ -Gly-Thr-J²¹-Glu-J²³-J²⁴-U²⁵-J²⁶-J²⁷-J²⁸-U²⁹-J³⁰-J³¹-Arg-J³³-J³⁴-J³⁵-J³⁶-J³⁷-Gln-J³⁹-U⁴⁰-J⁴¹-J⁴²-J⁴³-U⁴⁴-J⁴⁵-J⁴⁶-J⁴⁷-J⁴⁸-J⁴⁹-Arg-J⁵¹-U⁵²-J⁵³-J⁵⁴-Asp-U⁵⁶-Lys-Ser-Z⁵⁹-Leu-J⁶¹-Gly-J⁶³-J⁶⁴-Z⁶⁵-J⁶⁶-J⁶⁷-U⁶⁸-J⁶⁹-J⁷⁰-J⁷¹-J⁷²-J⁷³-J⁷⁴-Ser (II)

dans laquelle J, Z, U, X et B sont tels que définis ci-dessus.

Par exemple, J⁻² peut être Gly, J⁻¹ peut être Ser, et J⁰ peut être Cys, Thr, Pro, Ser, ou Gln. cette séquence J⁻²J⁻¹-J⁰ peut être choisie par exemple parmi Gly-Ser-Cys-, Gly-Ser-Thr-, Gly-Ser-Pro-, Gly-Ser-Ser-, Gly-Ser-Gly-, et Gly-Ser-Gln-. Ainsi, par exemple, chacune des séquences IDn°1 à IDn°10 précitée peut comporter au choix chacune des séquences fonctionnelles précitées. La séquence IDn°12 de la liste de séquences annexée n'est qu'un exemple non limitatif d'une séquence selon la présente invention comportant à son extrémité N-terminale une séquence fonctionnelle de trois acides aminés.

Selon un deuxième mode particulier de réalisation de la présente invention, les peptides peuvent comporter, par exemple à leur extrémité N-terminale, une séquence de fonctionnalisation de quatre acides aminés J⁻³-J⁻²J⁻¹-J⁰ choisie parmi Gly-Ser-Gly-Cys-, Gly-Cys-Gly-Ser, Gly-Ser-Gly-Ser, Gly-Cys-Gly-Cys, Gly-Cys-Gly-Ser. Cette séquence de fonctionnalisation est utile par exemple pour une fixation directe d'un marqueur tel que le technétium sur le peptide. Ce mode de réalisation est exposé ci-dessous. Ainsi, par exemple, chacune des séquences IDn°1 à IDn°10 précitées peut comporter au choix chacune des séquences fonctionnelles précitées. Les séquences IDn°11 de la liste de séquences annexée (plusieurs séquences sont regroupées en une seule sous la dénomination IDn°11) ne sont que des exemples non limitatifs de séquences selon la présente invention comportant à son extrémité N-terminale une séquence fonctionnelle de quatre acides aminés.

Selon un troisième mode particulier de réalisation de la présente invention, les peptides peuvent comporter, par exemple à leur extrémité N-terminale, une séquence de fonctionnalisation de sept à onze acides aminés. Cette séquence de fonctionnalisation est utile par exemple pour une fixation directe d'un marqueur tel que le technétium sur le peptide. Ce mode de réalisation est exposé ci-dessous. Ainsi, par exemple, chacune des séquences IDn°1 à IDn°10 précitées peut comporter au choix chacune des séquences fonctionnelles précitées. On peut aussi remplacer la séquence Gly-Ser-Gly-Cys des séquences IDn°11 à 14 par Gly-Bb1-Gly-Bb2, dans laquelle Bb1 et Bb2 sont indépendamment Cys ou Ser. Les séquences IDn°13 et 14 de la liste de séquences annexée (plusieurs séquences sont regroupées en une seule sous la dénomination IDn°13 ou 14) ne sont que des exemple non limitatifs de séquences selon la présente invention.

Les peptides de la présente invention ont une affinité suffisante pour le calcium et sont capables de se lier de manière réversible à des effecteurs lipidiques, et notamment à ceux chargés négativement, tel que les phosphatidylsérines, les acides phosphatidiques, les phosphatidyléthanolamines, les phosphatidylglycérols, les cardiolipines et les phosphatidylinositolphosphates.

Il s'agit d'une famille de peptides dont la propriété principale est de reconnaître spécifiquement l'apparition des signaux lipidiques à la surface des membranes cellulaires en relation avec le fonctionnement normal ou pathologique des tissus.

Les peptides de la présente invention peuvent être synthétisés par les procédés classiques de synthèse de la chimie organique ou de la chimie des protéines, ainsi que par recombinaison génétique *in vivo* ou *in vitro*, par génie génétique, etc.

Aussi, la présente invention se rapporte également à un procédé de fabrication d'un peptide selon l'invention, ledit procédé comprenant une synthèse chimique en phase solide dudit peptide. La synthèse chimique peut être réalisée par exemple avec un synthétiseur automatique de peptide du type Applied Biosystems, mod.433A. Elle peut être réalisée par exemple en chimie Fmoc qui utilise le groupement fluoremylméthyloxycarbonyle pour la protection temporaire de la fonction α-aminique des acides aminés.

Les éléments techniques pour la réalisation de ce procédé de synthèse peptidique sont connus de l'homme du métier. Ils sont décrits par exemple dans l'ouvrage Solid-Phase Organic Synthesis de Kevin Burgess (Editor) Wiley-Interscience; ISBN: 0471318256; (February 2000).

Le peptide de l'invention peut aussi être fabriqué par recombinaison génétique *in vivo* par exemple au moyen d'un procédé comprenant les étapes suivantes :
a) préparation d'un cDNA comprenant une séquence de base codant pour ledit peptide
b) insertion dudit cDNA dans un vecteur d'expression approprié,
c) transformation d'une cellule hôte appropriée, avec ledit vecteur dans lequel le cDNA a été inséré, pour une réplication du plasmide,
d) fabrication dudit peptide par traduction dudit cDNA dans ladite cellule hôte, et
e) récupération du peptide synthétisé.

Selon l'invention, le vecteur d'expression approprié et la cellule hôte sont choisis selon les techniques habituelles pour la recombinaison génétique. Le vecteur peut être l'un quelconque des plasmides généralement utilisés dans cette technique, par exemple un plasmide tel que le vecteur pGEX-2T. De même la cellule peut être choisie selon les techniques habituelles, il peut s'agir par exemple de *E*. *Coli*.

Lorsqu'une technique de recombinaison génétique *in vitro* est utilisée, les étapes c) et d) du procédé ci-dessus sont remplacées respectivement par les étapes c') d'introduction du vecteur dans lequel le cDNA a été inséré dans un milieu réactionnel adéquat pour une réplication du plasmide, et d') de fabrication dudit peptide par traduction dudit cDNA dans ledit milieu réactionnel adéquat. Le document Jagus, R. and Beckler, G.S. (1998) Overview of eukaryotic in vitro translation and expression systems, Current Protocols in Cell Biology 11.1.1-11.1.13., 1998 by John Wiley & Sons, Inc. décrit des procédés *in vitro* utilisables dans la présente invention.

La présente invention fournit également un assemblage chimique ayant une affinité pour un phospholipide, comprenant au moins deux peptides de la présente invention, identiques ou différents, lesdits peptides étant liés entre eux. Ces assemblages peuvent être réalisés par exemple par insertion d'un lien peptidique flexible, par exemple poly-glycine, entre le résidu C-terminal d'un peptide de l'invention et le résidu N-terminal du second peptide et ainsi de suite selon le nombre de peptides mis bout à bout. Ce lien poly-glycine peut être de formule -(Gly)ₙ-, n étant un nombre entier allant de 1 à 12, par exemple supérieur à 4. Selon l'invention, au moins un des peptides de l'assemblage peut être un peptide comportant une séquence choisie parmi les séquences ID n°1 à 10 de la liste de séquences annexées.

Ces assemblages peuvent également être synthétisés par les procédés classiques de synthèse de la chimie organique ou de la chimie des protéines, ainsi que par recombinaison génétique *in vivo* ou *in vitro*, par génie génétique, etc., par exemple par un des procédés précités.

Ces assemblages ont notamment pour but d'augmenter l'affinité des peptides de la présente invention pour le phospholipide, par exemple pour un phospholipide chargé négativement.

L'utilisation d'un assemblage de la présente invention peut se faire dans trois directions : la thérapeutique, la recherche et le diagnostic, et les applications sont très nombreuses.

Les pathologies spécialement visées par la présente invention sont : (i) les troubles de la coagulation sanguine, (ii) les phénomènes d'apoptose consécutifs à l'action de composés chimiques, d'effets physiques comme les radiations ionisantes, d'effets biologiques comme ceux liés à la formation ou la nécrose des tissus cancéreux, outre les phénomènes normaux d'apoptose, (iii) les pathologies inflammatoires, et (iv) les troubles associés aux relations entre les cellules et la matrice extracellulaire et notamment le collagène.

Les peptides de la présente invention présentent en outre un avantage important par rapport aux composés de l'art antérieur : la réversibilité de leurs processus de repliement qui permet leur manipulation à des températures élevées mais compatibles avec la stabilité chimique des peptides, à des fins de modifications chimiques dans le but de développer des molécules utilisables en imagerie et en thérapeutique.

En outre, en raison de leur faible taille, les peptides de la présente invention peuvent être facilement associés à d'autres protéines soit pour former des protéines chimères multifonctionnelles, soit pour introduire un mécanisme de régulation par des effecteurs autres que les phospholipides de signalisation.

Les peptides de la présente invention peuvent être utilisés en tant que tels pour fabriquer un médicament utilisable pour un traitement ou pour une prophylaxie car ils possèdent des propriétés anticoagulante et anti-inflammatoire intrinsèques. Ils permettent d'effectuer un tapissage des surfaces cellulaires, capable d'interdire l'accès de composés impliqués dans les étapes primaires de la coagulation sanguine et les phénomènes inflammatoires à ces surfaces.

Ainsi, selon l'invention, les peptides ou assemblages de la présente invention peuvent être utilisés en tant que tels pour préparer un médicament, par exemple choisi parmi un médicament destiné au traitement d'une thrombose, un médicament destiné au traitement d'une tumeur, un médicament ayant une action anti-inflammatoire.

Les peptides de la présente invention peuvent également être utilisés couplés à des molécules de traitement pour le ciblage de ces molécules vers des zones présentant des charges négatives telles qu'à un site du thrombus, d'une inflammation ou vers une zone tumorale. Dans cette application, les peptides et assemblages de la présente invention sont par exemple couplés respectivement à une molécule qui a une action thrombolytique, à une molécule qui a une action anti-inflammatoire ou à une molécule qui a une action antitumorale. Des exemples de molécules ayant une activité thrombolytique utilisable selon la présente invention sont les streptokinases, les urokinases, et les activateurs du plasminogène. D'une façon générale les peptides et assemblages de la présente invention peuvent être couplés indifféremment à des composés pro-apoptotiques anti-apoptotiques et anti-inflammatoires.

Les peptides et assemblages de la présente invention peuvent donc être utilisés couplés à une molécule ayant une activité thrombolytiques pour fabriquer un médicament utilisable dans le traitement et la prophylaxie de la thrombose ; couplés à une molécule ayant une action anti-inflammatoire pour fabriquer un médicament utilisable par exemple par voie locale ou par voie intraveineuse pour traiter des pathologies aiguës comme l'asthme, la RCH, la maladie de Crohn, le choc septique, les maladies du collagène et l'arthrite ; ou couplés à une molécule ayant une action antitumorale pour fabriquer un médicament utilisable pour traiter des tumeurs.

Pour une utilisation dans la recherche ou le diagnostique, les peptides de la présente invention peuvent être couplés à une molécule de marquage permettant sa détection. Cette molécule de marquage peut être par exemple une molécule fluorescente, des particules d'or ou de composés denses, de type nanoparticules, pour la microscopie électronique, un radioélément, un composé paramagnétique et de manière générale, une des molécules de marquage couramment utilisées dans les laboratoires. Pour faciliter certains marquages ou liaisons, cette molécule pourra être liée à l'un des partenaires du système avidine-biotine.

Selon l'invention, les peptides et les assemblages chimiques selon l'invention peuvent être couplés à une molécule de marquage pour former un composé de marquage utilisable par exemple pour un diagnostique in vivo ou *in vitro*.

En effet, les peptides de la présente invention peuvent être utilisés pour la détection de pathologies impliquant l'apparition de charges négatives à la surface des cellules et la libération dans le sang de microvésicules: par exemple les troubles de la coagulation, les pathologies inflammatoires aiguës, etc. et l'apoptose.

Ils peuvent, par exemple, être couplés à des radioéléments à vie courte tels que le technétium ou le fluor 18 et permettre une détection "*in vivo*" de la localisation des zones thrombotiques lors d'accidents vasculaires de toutes sortes, en particulier des foyers apoptotiques et inflammatoires, en utilisant des systèmes d'imagerie.

Les peptides de la présente invention peuvent aussi, par exemple, être couplés à des composés paramagnétiques, tel qu'un complexe de gadolinium, à tout agent de contraste utilisable en imagerie par résonance magnétique (IRM) tel que par exemple un composé paramagnétique ou un composé ferromagnétique, ou à tout élément radioactif à durée de vie courte. Ils peuvent ainsi permettre une détection "*in vivo*" de la localisation des zones thrombotiques, des zones apoptotiques et inflammatoires.

Les couplages précités peuvent être réalisés par toutes les techniques classiques de chimie organique connues de l'homme du métier.

Par exemple dans le cas du technétium, ce dernier peut être couplé directement au peptide de la présente invention, par exemple lorsque le peptide de l'invention comporte une séquence de fonctionnalisation telles que celles décrites ci-dessus. Ce type de couplage est décrit par exemple dans le document US 6 323 313 de J. F. Tait et al. L'homme du métier comprendra que des marqueurs équivalents au technétium pourront également être couplés de cette manière directement au peptides de la présente invention.

Le technétium, ou tout autre métal tels que ceux cités dans la présente, peut aussi être couplé indirectement au peptide de la présente invention. Ce couplage est réalisé par exemple au moyen d'une cage complexant ledit métal. Cette cage peut être fixée au peptides de la présente invention au moyen d'une séquence de fonctionnalisation telles que celles décrites ci-dessus. Dans l'exemple du technétium, des cages à technétium utilisables selon la présente invention sont décrites par exemple dans le document 99mTc Labeling of Highly Potent Small Peptides Shuang Liu, D. Scott Edwards, and John A. Barrett, Bioconjugate Chem. 1997, 8, 621-636.

Les peptides ou assemblages couplés ou prêts à être couplés, suivant l'application désirée, peuvent être avantageusement conditionnés sous la forme de trousses de diagnostique. Ainsi, la présente invention fournit également une trousse de diagnostic comprenant un peptide ou un assemblage conformes à la présente invention. Cette trousse de diagnostic peut par exemple comprendre en outre un réactif adéquat permettant de détecter ladite molécule de marquage.

La présente invention fournit également une trousse d'analyse et de détection de charges négatives à la surface de cellules, caractérisée en ce qu'elle comprend un peptide ou un assemblage chimique de la présente invention, le peptide ou l'assemblage pouvant être couplé à un marqueur.

La présente invention fournit également une trousse d'analyse et de détection de microvésicules dans le sang, caractérisée en ce qu'elle comprend un peptide ou un assemblage chimique conformes à la présente invention, le peptide ou l'assemblage pouvant être couplé à un marqueur.

Dans une autre application de la présente invention, des peptides ou des assemblages de l'invention peuvent être utilisés, par exemple, pour le recouvrement d'un matériau biocompatible. Ce type de matériau est utilisable dans deux sortes de conditions : i) les circulations extracorporelles, et ii) la conservation du sang.

Ainsi, les peptides de la présente invention trouvent une application par exemple pour la fabrication d'un filtre pour le piégeage et la récupération, dans la circulation sanguine extra corporelle de cellules circulantes activées : plaquettes, globules rouges, leucocytes... Le sang réintroduit chez le patient sera ainsi débarrassé des cellules susceptibles de créer des coagulations anormales, des réactions fébriles etc.... Ce filtre peut se présenter sous la forme d'un film plissé de polymère biocompatible sur lequel peuvent être greffé par tout moyen approprié les peptides de la présente invention. Ces mêmes filtres peuvent être introduits dans les poches servant à la conservation du sang ou tapisser l'intérieur de celles-ci. Ces filtres constituent des "éponges" capable de capter en continu les cellules sanguines contenues dans la poche qui sont activées en particulier à la suite de leur vieillissement et de leur engagement dans le processus d'apoptose.

Les différents marquages, couplages et fixation exposés ci-dessus, se feront de toute préférence en préservant l'activité du peptide de la présente invention, en général aux extrémités ou au niveau des extrémités du peptide de la présente invention ou sur des résidus de surface.

D'autres avantages et caractéristiques de la présente invention apparaîtront encore à la lecture des exemples illustratifs et non limitatifs qui suivent, en référence aux figures en annexe.

### BREVE DESCRIPTION DE LA LISTE DE SEQUENCES

- Les séquences IDn°1 à IDn°14 annexées sont des exemples de peptides comportant la séquence peptidique de la revendication 1 de la présente invention.

En, particulier, les séquences IDn°11, IDn° 13 et IDn°14 sont des exemples de peptides comportant la séquence peptidique de la présente invention dans lesquels des mutations ont été introduites pour augmenter l'affinité pour le calcium et les phospholipides.

### BREVE DESCRIPTION DES FIGURES

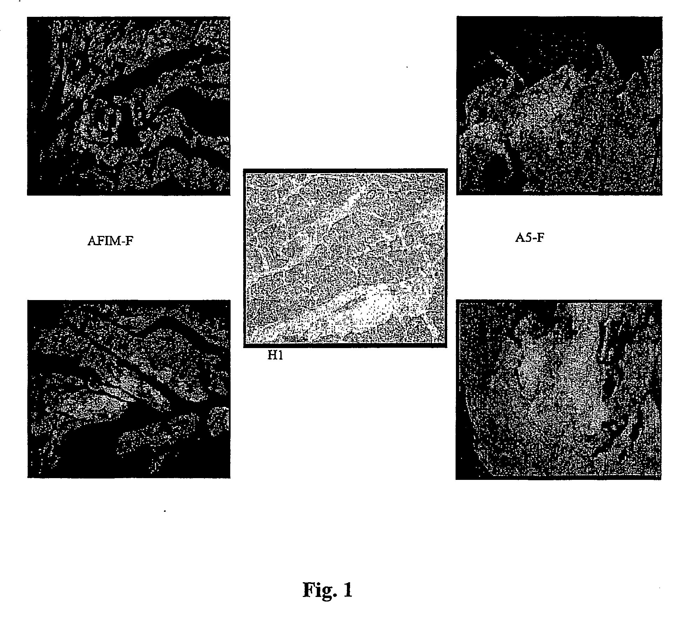

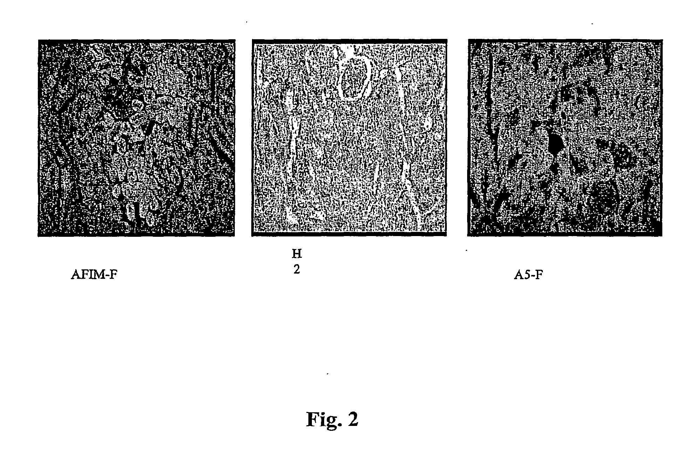

- Les figures 1 et 2 sont des micrographies obtenues à partir de coupes de tissus respectivement d'un coeur apoptotique (figure 1) et d'un rein (figure 2). Ces coupes ont été obtenues d'une part (clichés de gauche) avec des peptides AFIM-fluorescéine (AFIM-F) de la présente invention, d'autre part (clichés de droite) avec de l'annexine 5 -fluorescéine (A5-F) (composé de l'art antérieur) : microscopie de fluorescence, grossissement x 40. Les clichés du centre ont été obtenus avec de l'hématoxyline : microscope en lumière visible, grossissement x 40. Sur la figure 1, les photos du haut et du bas représentent différentes coupes du coeur.

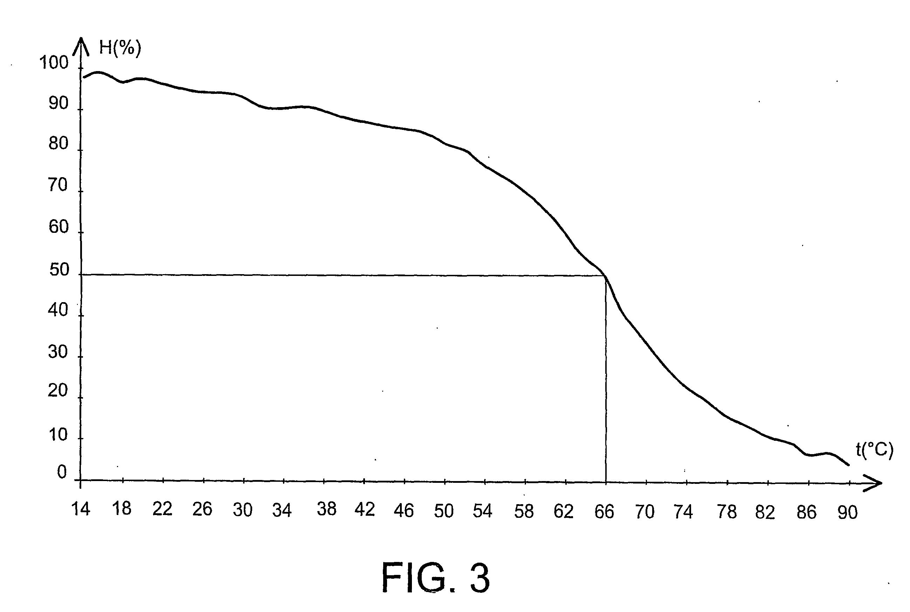

- La figure 3 est un graphique qui représente le taux d'hélicité « H » (en %) d'un peptide selon la présente invention en fonction de la température « t » en °C.

### EXEMPLES

### Exemple 1: Synthèse par recombinaison génétique: Expression et purification des peptides de séquences ID n°1 à ID n°12 de la présente invention

Les séquences ID n°1 à ID n°14 ont été préparées par surexpression dans *E*. *Coli* selon le même protocole que celui qui a été décrit par F. Cordier-Ochsenbein et al. dans J. Mol. Biol. 279, 1177-1185.

Les cDNA de chacune de ces séquences ont été préparés en utilisant une réaction de polymérase en chaîne (PCR). Ils ont été insérés dans le vecteur pGEX-2T (Smith & Jonhson, 1998). La figure 2 est un schéma illustrant l'insertion du cDNA dans le vecteur. L'absence de mutations induites par la PCR a été contrôlée par séquençage.

La production des peptides est effectuée en utilisant la souche *E*. *Coli* BL21 contenant le vecteur d'expression décrit plus haut. Après induction par l'isopropylthiogalactopyranoside (IPTG, 100 µm) jusqu'à une densité optique de 1 à 600 nm, la pousse est continuée jusqu'à ce qu'un plateau soit atteint, c'est-à-dire pendant environ 3 heures. Après centrifugation, les bactéries sont re-suspendues dans le tampon de lyse comprenant 50 mM Tris-HCl, pH 8, 10 mM EDTA, 500 mM NaCl, 5% (v/v) glycérol, 1% (v/v) Triton X100, 1 mM dithiothréitol (DTT), 1 mM fluorure de phénylméthylsulfonyl (PMSF) et 20 µg/ml d'aprotinine.

La purification a été effectuée de la façon suivante: après sonication et centrifugation à 10000 g, le surnageant contenant les protéines solubles est incubé avec des billes de glutathion/agarose permettant la liaison spécifique à ces billes de la protéine de fusion GST-domaine. Après lavage avec une solution contenant 1 M NaCl, 50 mM Tris-HCl à pH 8, 70 unités de thrombine par litre de culture sont ajoutés et les séquences sont éluées.

Les séquences sont alors purifiées sur une colonne proRPC (marque de commerce) de type 16/10, fournie par la société Pharmacia en utilisant un système FPLC et un gradient linéaire d'eau de qualité Millipore (marque de commerce) contenant 0,1% (v/v) d'acide trifluoroacétique TFA, et d'acétonitrile contenant 0,1% de TFA. La vitesse d'écoulement est ajustée à 2,5 ml/minute. Les séquences sont ensuite lyophilisées.

Le rendement final pour chaque peptide est d'environ 8 mg de séquence par litre de culture.

### Exemple 2 : Exemple de synthèse chimique de peptides de la présente invention

Les peptides de la présente invention ont été fabriqués dans cet exemple par synthèse chimique en phase solide avec un synthétiseur automatique de peptides Applied Biosystems, mod. 433A, et en chimie Fmoc, qui utilise le groupement Fluorénylméthyloxycarbonyle (Fmoc) pour la protection temporaire de la fonction α-aminique des acides aminés.

Les groupements protecteurs utilisés pour prévenir les réactions secondaires des chaînes latérales des acides aminés, dans cette stratégie Fmoc, ont été le tertio-butyle éther (tBu) pour les résidus Ser, Thr et Tyr ; tertio-butyle ester (OtBu) pour Asp, Glu; trityle (Trt) pour Gln, Asn, Cys, His ; tertio-butyloxycarbonyle (Boc) pour Lys et 2,2,5,7,8-pentamétylchromane-6-sulfonyle (Pmc) pour Arg.

La réaction de couplage se déroule avec un excès de 10 équivalents d'acides aminés (1 mmol) par rapport à la résine (0,1mmol). L'acide aminé protégé est dissous dans 1 ml de N-méthylpyrollidone (NMP) et 1 ml d'une solution de 1-N-hydroxy-7-azabenzotriazole (HOAt) 1M dans le solvant NMP. 1 ml d'une solution de N,N'-dicyclohexylcarbodiimide (DCC) 1M est alors ajouté. Après 40 à 50 minutes d'activation, l'ester actif formé est transféré dans le réacteur qui contient la résine. Avant cette étape de transfert puis de couplage, la résine est déprotégée de son groupement Fmoc par une solution de 20 % de pipéridine dans le NMP. L'excès de pipéridine est enlevé par lavage à la NMP après 5 à 10 minutes environ.

Pendant la déprotection, la détection des adduits dibenzofulvène-pipéridine à 305 nm permet de suivre le bon déroulement de la synthèse. En effet, la quantification de l'adduit permet d'estimer l'efficacité de la déprotection du groupement Fmoc et par suite du couplage du dernier acide aminé incorporé.

Le clivage de la résine et des groupements protecteurs présents sur les chaînes latérales a été réalisé simultanément par traitement du peptide lié à la résine par de l'acide trifluoroacétique (TFA). Avant d'effectuer le clivage, la résine a été lavée plusieurs fois au dichlorométhane (DCM) et enfin séchée. Le réactif utilisé lors du clivage est un mélange acide contenant 81,5 % de TFA et les piégeurs phénol (5 %), eau (5 %), éthanedithiol (2,5% lorsque le peptide comporte une cystéine) et tri-isopropylsilane (1 %). La résine a été traitée avec ce mélange pendant trois heures sous agitation et à température ambiante, à raison de 100 ml de solution par gramme de résine. Le peptide libre en solution a été récupéré par filtration. Le peptide a été ensuite précipitée et lavée à froid dans l'éther de diisopropyle puis dissous dans de l'acide acétique à 20 % et lyophilisée.

Le peptide récupéré après lyophilisation, le brut de synthèse, se trouve sous forme réduite, c'est à dire que les ponts disulfures inter-chaînes ne sont pas formés.

Le peptide est alors purifié sur une colonne proRPC (marque de commerce) de type 16/10, fournie par la société Pharmacia en utilisant un système FPLC et un gradient linéaire d'eau de qualité Millipore (marque de commerce) contenant 0,1% en volume d'acide trifluoroacétique TFA, et d'acétonitrile contenant 0,1% de TFA. La vitesse d'écoulement est ajustée à 2,5 ml/minute. Le peptide est ensuite lyophilisé.

Les produits obtenus ont été analysés par spectrométrie de masse.

### Exemple 3 : Stabilité des séquences ID n°1 à IDn°14

Cet exemple montre que les peptides de la présente invention constituent des protéines de repliement stables.

### Composition du blanc (témoin):

| | |
|---|---|
| Tris 50 mM, NaCl 150 mM, DTT 1 mM pH8 | 10 µL |
| H₂O | 990 µL |
| Ajusté à pH8 | |

### Composition de l'échantillon :

| | |
|---|---|
| Echantillon : | domaine purifié dans tampon Tris 50 mM, NaCl 150 mM, pH8 Concentration aprox. : 200 mg.ml. |
| Domaine: | 10 µL soit 300 µM final. |
| H₂O : | 990 µL. |
| pH mesuré à 7,8. | |

### Configuration matérielle et logicielle :

Appareil Jobin Yvon CD6.
Logiciel CD-max
Trajet optique de la cuvette de mesure : 1 cm.

La figure 1 annexée représente le taux d'hélicité de AFIM en fonction de la température tel qu'il est mesuré à l'aide du signal de dichroïsme circulaire dans l'UV lointain à la longueur d'onde de 220 nm.

Sur cette figure, la valeur du signal à 14°C est prise pour le 100% du contenu en hélice du peptide. La dénaturation thermique du peptide est bien coopérative et démontre qu'à basse température et notamment à 37°C il s'agit d'un peptide convenablement replié et présentant une stabilité améliorée.

### Exemple 4 : Assemblages de deux peptides de la présente invention

Le procédé décrit dans l'exemple 1 ci-dessus est utilisé pour synthétiser une séquence peptidique de séquence IDn°1-(gly)₄-IDn°1.

Le rendement final pour l'assemblage est d'environ 14 mg/litre de culture.

### Exemple 5 : Marquage d'un peptide de la présente invention par la fluorescéine

Dans les exemples qui suivent, le peptide de la présente invention est appelé AFIM-SH. Il a une séquence peptidique telle que définie par la séquence (I). Les séquences IDn°1 à IDn°14 sont testées.

La fluorescéine est une molécule qui émet une fluorescence verte d'une longueur d'onde de 525 nm lorsqu'elle est excitée à une longueur d'onde de 488 nm. L'émission de lumière verte est détectée par des caméras ou des photomultiplicateurs. Ce couplage d'AFIM à la fluorescéine permet de détecter la présence des cellules présentant la PS aussi bien *in vitro* que in vivo chez des petits animaux.

Selon la présente invention, il est possible de marquer AFIM au niveau des résidus de surface sur toute cystéine qui serait introduite à la place de n'importe quel acide aminé présent à la surface d'AFIM (résidus de surface) pour autant que la fonction de liaison aux membranes lipidiques ne soit pas perturbée. AFIM ainsi modifié est désigné AFIM-SH ci-dessous.

Le couplage de la fluorescéine se fait par l'intermédiaire d'une fonction maléimide représentée ci-dessous sur AFIM par la fonction SH.

La fluorescéine est couplée à une ou plusieurs cystéine(s) de la séquence, de manière covalente, en utilisant une fonction maléimide.

### Schéma général du marquage (schéma I):

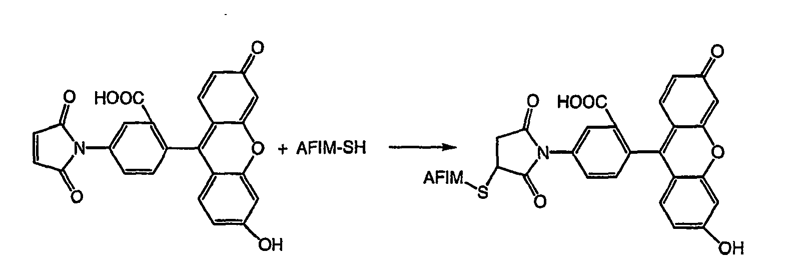

Tout le marquage se fait à une température inférieure à 20°C.

AFIM-SH est en solution dans du tampon Tris (50 mM), NaCl (150 mM), pH = 7.4. 5 équivalents de DTT en solution dans le même tampon sont additionnés à la solution de AFIM-SH. Le milieu est agité durant 30 min.

A l'abri de la lumière: la fluorescéine (5 équivalents d'AFIM-SH + 2 équivalents de DTT) est pesée et dissoute dans du DMF, et additionnée à la solution précédente. Le tout est agité, et la réaction est poursuivie 30 min. Puis le milieu est dilué dans 150 ml de tampon PBS (Phosphate 20 mM, NaCl 150 mM), pH = 7.4, et ultra-filtré sur membrane YM3 (marque de commerce). L'échantillon est re-dilué et ultra-filtré plusieurs fois, en faisant le spectre UV du filtrat.

Lorsqu'il n'y a plus de fluorescéine dans le filtrat (pic à 490 nm), l'échantillon est concentré à quelques ml et conservé au frais à 4°C.

Les produits AFIM-Fluorescéine ont été employés pour détecter des cellules apoptotiques en cytométrie de flux *in vitro,* ainsi que chez des animaux *in vivo* de la manière décrite dans l'exemple 6 suivant.

### Exemple 6 : Résultats de marquages de cellules apoptotiques par les produits AFIM-Fluorescéine

### Imagerie de cellules cardiaques apoptotiques après un infarctus chez le rat.

Un modèle d'apoptose chez le rat est utilisé comme il est décrit dans l'article paru dans Circulation Res. 1996, 79, 946-956.

Brièvement, quatre rats (300 g chacun) ont été anesthésiés, intubés et ventilés. L'ischémie du myocarde fut provoquée par une occlusion temporaire de l'artère coronaire. Après 30 minutes d'occlusion, l'artère coronaire fut re-perfusée pendant une heure.

A la fin de la période de re-perfusion, les peptides AFIM-Fluorescéine de l'exemple 5 ont été injectés dans la veine jugulaire à raison de 200 µg de peptide pour chacun de deux des rats dans un volume total de 1 ml.

A titre comparatif, 200µg d'annexine 5-Fluorescéine (composés de l'art antérieur) ont été injectés dans les mêmes conditions pour chacun des deux autres rats dans un volume total de 1 ml.

Les rats ont été sacrifiés après 60 minutes.

Cinq organes ont été conservés pour cette étude: le coeur, le poumon, le rein, le foie et le cerveau. Il ont été lavés et rincé en présence de formol. Les organes ont ensuite été déshydratés et imprégnés de paraffine pendant environ 12 heures puis des coupes de 7 µm furent effectuées.

Quelques coupes furent colorées à l'hématoxyline. Les coupes ont été examinées au microscope à fluorescence et les coupes adjacentes colorées à l'hématoxyline furent examinées avec un microscope en lumière visible. Les coupes colorées à l'hématoxyline (marquées H1 et H2 respectivement sur les figures 1 et 2 annexées) permettent une visualisation de l'architecture des tissus et la microscopie de fluorescence de détecter le marquage par AFIM-Fluorescéine (AFIM-F) ou par l'annexine 5-Fluorescéine (A5-F).

La figure 1 annexée montre les images obtenues pour le coeur apoptotique et la figure 2 annexée montre les images obtenues pour le rein.

La figure 1 montre clairement l'excès de fluorescence correspondant à l'accumulation de marqueur au niveau des cellules apoptotiques. Le contraste est visiblement bien meilleur avec AFIM de la présente invention qu'avec l'annexine 5 de l'art antérieur.

La figure 2 montre le marquage du rein lié à l'élimination partielle des produits. Dans le cas de AFIM les glomérules ne semble pas marqués, seule les tubules proximaux sont partiellement marqués. Par contre dans le cas de l'annexine 5 de l'art antérieur l'ensemble du tissus rénal est fortement marqué, ce qui est en accord avec la toxicité rénale observée pour cette protéine.

Les résultats obtenus dans cet exemple démontrent une grande spécificité des peptides de la présente invention pour le marquage des cellules.

Le marquage du peptide AFIM, par exemple de IDn°1 à 10, par la fluorescéine permet donc de détecter efficacement la phosphatidylsérine (PS) présente à la surface externe des cellules impliquées dans des processus physiopathologiques comme la mort cellulaire programmée (apoptose) la coagulation du sang, la réaction inflammatoire.

### Exemple 7 : Marquage d'un peptide de la présente invention par le technétium ^{99m}Tc

Le marquage d'AFIM par le ^{99m}Tc permet, comme pour la fluorescéine, de détecter la phosphatidylsérine (PS) présente à la surface externe des cellules impliquées dans des processus physiopathologiques comme la mort cellulaire programmée (apoptose), la coagulation du sang, la réaction inflammatoire. Le ^{99m}TC est un émetteur de rayons γ qui permet une détection d'AFIM dans toute zone du corps par différents types de caméras détectant les rayonnements γ. Ce couplage d'AFIM au ^{99m}Tc permet de détecter la présence des cellules présentant la PS in vivo chez tout être vivant.

Deux types de marquage au technétium sont exposés dans cet exemple : un marquage indirect (A) et un marquage direct (B).

### A) MARQUAGE INDIRECT

Dans cet exemple, AFIM-SH est couplé, au niveau d'une cystéine de sa séquence, à une molécule complexante appelée molécule cage, capable de recevoir de manière spécifique l'ion ^{99m}Tc. La réaction de couplage est schématisée ci-dessous (schéma II).

La molécule cage choisie est le NH₂-C₃(Bham)₂ (2) décrit dans le document suivant: Bis(Hydroxamamide)-Based Bifunctional Chelating Agent for 99mTc Labelling of Polypeptides, Le-Cun Xu et al. Bioconjugate Chem. 1999, 10, 9-17. Cette cage est couplée au dérivé maléimide (1) selon le schéma (II) pour donner le marqueur (3) qui est ensuite couplé à AFIM-SH pour donner le composé nommé AFIM-cage (Schéma (II)). Le couplage est effectué de la façon suivante:

AFIM-SH est en solution dans du tampon Tris (50 mM), NaCl (150 mM), pH = 7.4. 5 équivalents d' hydrochlorure de tris-(2-carboxyéthyl)-phosphine (TCEP) sont pesés, dissous dans le même tampon et additionnés à AFIM-SH. Le milieu est agité et laissé à température ambiante durant 30 minutes.

Pendant ce temps, 10 équivalents de (1) sont mis en solution dans du diméthylformamide (DMF) et transférés sur 20 équivalents de cage (2) dans le même volume de DMF. Après 10 min de réaction, le produit est ajouté à AFIM-SH.

Le tout est agité, et la réaction est poursuivie 30 min à température ambiante. Puis le milieu est dissous dans 150 ml de tampon Tris (50 mM), NaCl (150 ml), pH = 7.4, et ultra-filtré sur membrane YM3 (marque de commerce). L'échantillon est re-dilué et ultra-filtré plusieurs fois, en faisant le spectre UV du filtrat. Lorsqu'il n'y a plus de DMF dans le filtrat (pic à 214 nm), l'échantillon est concentré à quelques ml et conservé au frais (4°C).

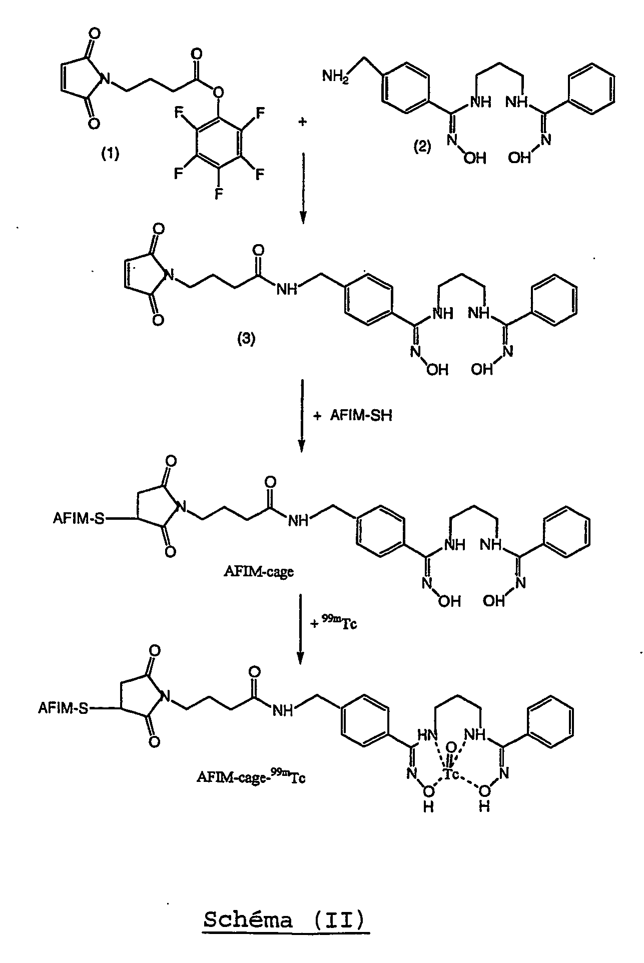

Une quantité adaptée à la taille de l'animal, du peptide couplé à la cage à technétium (AFIM-cage) préparé dans cet exemple, est prélevée et une solution aqueuse de SnCl₂ (6 équivalents par rapport au peptide) est ajoutée. La solution de ^{99m}TcO₄⁻ est ajoutée et la réaction est poursuivie 30 minutes à température ambiante.

La solution de peptide marqué (AFIM-cage-^{99m}Tc) est ensuite directement injectée par voie intraveineuse à l'animal.

Les images sont alors recueillies au moyen d'une caméra capable de détecter des rayons γ (SPECT ou autre caméra).

### B) MARQUAGE DIRECT

Dans cet exemple, AFIM est marqué au technétium sans cage. Pour cela, AFIM est doté d'une séquence de fonctionnalisation de quatre acides aminés qui fixent directement le technétium.

La séquence peptidique IDn°11 est utilisée dans cet exemple. La séquence de fonctionnalisation est Gly-Ser-Gly-Cys du côté N-terminal, les résidus 5 à 79 de la séquence IDn°11 étant ceux formant le séquence (I) de la présente invention.

Pour le marquage, le peptide de séquence IDn°11 et 5 équivalents de TCEP sont mis en solutions dans du sérum physiologique et équilibrés pendant 15 mn. On ajoute ensuite 10 équivalents de SnCl₂. Cette solution peut être lyophilisée et conservée sous azote.

Le marquage est effectué en ajoutant une solution de ^{99m}TcO₄⁻. Après une incubation de 15 minutes, la solution est passée sur une colonne PD10 (marque de commerce).

La séquence IDn°11 marquée directement au technétium (AFIM-^{99m}Tc) est injectée par voie intraveineuse.

Les images sont alors recueillies avec une caméra telle que celle utilisée ci-dessus.

### Exemple 8 : Marquage d'un peptide de la présente invention avec du gadolinium

### AFIM couplé au gadolinium : AFIM-cage-Gd (marquage indirect).

Le marquage d'AFIM par le gadolinium permet, comme pour les précédents marqueurs, de détecter la phosphatidylsérine (PS) présente à la surface externe des cellules impliquées dans des processus physiopathologiques comme la mort cellulaire programmée (apoptose), la coagulation du sang, la réaction inflammatoire. Le gadolinium est un agent paramagnétique qui permet une détection d'AFIM dans toute zone du corps par des procédés d'imagerie par résonance magnétique nucléaire. Ce couplage d'AFIM au gadolinium permet de détecter avec une résolution pouvant aller jusqu'au mm la présence des cellules présentant la PS in vivo chez tout être vivant.

Comme pour la fluorescéine, AFIM peut être couplée, au niveau d'une cystéine, à une molécule chimique capable de recevoir de manière spécifique l'ion gadolinium. Une fois cette cage à gadolinium construite, le couplage est réalisé avec AFIM comme décrit ci-dessous.

AFIM-SH est en solution dans du tampon Tris (50 mM), NaCl (150 mM), pH = 7.4. 5 équivalents de TCEP sont pesés et dissous dans le même tampon et additionnés à AFIM-SH. Le milieu est agité et laissé à T.A. durant 30 min.

La cage à gadolinium utilisée est celle décrite dans le document P. KANTHI, et al. ; « Synthesis of Charged and Uncharged Complexes of Gadolinium and Yttrium with Cyclic Polyazaphosphinic Acid Ligands for in vivo Applications », J.CHEM SOC. PEKIN TRANS. 2, 1993, pp.605-618.

5 équivalents de cage, par rapport à AFIM-SH, sont dissoutes dans du DMF, et ajoutés à AFIM-SH. Le tout est agité, et 1a réaction est poursuivie 30 min à T.A. Puis le milieu est dissous dans 150 ml de tampon Tris (50 mM), NaCl (150 nM), pH = 7.4, et ultra-filtré sur membrane YM3. L'échantillon est re-dilué et ultra-filtré plusieurs fois, en faisant le spectre UV du filtrat. Lorsqu'il n'y a plus de DMF dans le filtrat (pic à 214 nm), l'échantillon est concentré à quelques ml et conservé au frais à 4°C.

Le produit, une fois purifié est injecté par voie intraveineuse.

Les images sont recueillies au moyen d'une caméra de détection.

### Exemple 9 : Marquage d'un peptide de la présente invention avec de l'or

Le marquage d'AFIM par l'or est un marquage direct. Il permet de détecter la phosphatidylsérine (PS) présente à la surface externe des cellules impliquées dans des processus physiopathologiques comme la mort cellulaire programmée (apoptose) la coagulation du sang, la réaction inflammatoire.

L'or est un métal dense aux électrons et permet son usage en microscopie électronique. Ce couplage d'AFIM à l'or permet de détecter et de localiser la phosphatidylsérine à l'échelle de la cellule et des compartiments sub-cellulaires. Le produit couplé peut être utilisé *in vitro.*

AFIM-SH est mis en solution dans du tampon Tris (50 mM), NaCl (150 mM), pH = 7.4. 5 équivalents de tris-(2-carboxyéthyl)-phosphine (TCEP) en solution dans le même tampon sont additionnés à AHM-SH. Le milieu est agité durant 15 min.

Des billes d'or modifiées (contenant un maléimide greffé: Nanogold Monomaléimide Interchim (marque déposée)) sont dissoutes dans 20 µl de DMSO et 200 µl d'eau ; et additionnées à la solution précédente (2 équivalents de billes par rapport à la protéine).

Le tout est agité, et la réaction est poursuivie une heure. Puis le milieu est purifié sur colonne de gel filtration (PD-10 Pharmacia (marque de commerce)) et élué avec du tampon PBS (Phosphate 20 mM, NaCl 150 mM), pH = 7.4

AFIM-Au est utilisable sur des coupes de tissus ou sur des cellules isolées. L'analyse peut se faire par microscopie électronique.

## Revendications

1. Peptide **caractérisé en ce qu'**il est constitué de la séquence peptidique suivante :
J¹-J²-J³-J⁴-J⁵-J⁶-Asp-U⁸-J⁹-J¹⁰-U¹¹-Arg-J¹³-Ala-U¹⁵-Lys-Gly-X¹⁸-Gly-Thr-J²¹-Glu-J²³-J²⁴-U²⁵-J²⁶-J²⁷-J²⁸-U²⁹-J³⁰-J³¹-Arg-J³³-J³⁴-J³⁵-J³⁶-B³⁷-Gln-J³⁹-U⁴⁰-J⁴¹-J⁴²-J⁴³-U⁴⁴-J⁴⁵-J⁴⁶-J⁴⁷-J⁴⁸-J⁴⁹-Arg-J⁵¹-U⁵²-J⁵³-J⁵⁴-Asp-U⁵⁶-Lys-Ser-Z⁵⁹-Leu-J⁶¹-Gly-J⁶³-J⁶⁴-Z⁶⁵-J⁶⁶-J⁶⁷-U⁶⁸-J⁶⁹-J⁷⁰-J⁷¹-U⁷²-J⁷³-J⁷⁴-Ser
dans laquelle J, Z, U, X et B représentent des acides aminés tels que :
- les acides aminés J sont choisis indépendamment les uns des autres parmi les acides aminés naturels, ou des dérivés de ceux-ci, de telle manière qu'au moins 50% d'entre eux sont des résidus polaires choisis parmi Arg, Asn, Asp, Cys, Gln, Glu, Gly, His, Lys, Orn, Pro, Ser, Thr et Tyr,
- l'acide aminé X¹⁸ est choisi indépendamment des autres acides aminés de la séquence parmi Ala, Asn, Cys, Gln, Gly, His, Ile, Leu, Met, Phe, Ser, Thr, Trp, Tyr et Val,
- les acides aminés Z⁵⁹ et Z⁶⁵ sont choisis indépendamment parmi Glu, Asp, Lys ou Arg,
- les acides aminés U et B de la séquence sont choisis suivant un des exemples a) à j) exposés dans le tableau 1 suivant :
| | **U⁸** | **U¹¹** | **U¹⁵** | **U²⁵** | **U²⁹** | **B³⁷** | **U⁴⁰** | **U⁴⁴** | **U⁵²** | **U⁵⁶** | **U⁶⁸** | **U⁷²** |
|---|---|---|---|---|---|---|---|---|---|---|---|---|
| **Ex a)** | Val | Leu | Met | Ile | Leu | Arg | Ile | Tyr | Leu | Leu | Val | Leu |
| **Ex b)** | Ala | Ile | Ile | Ile | Leu | Arg | Ile | Tyr | Leu | Leu | Ile | Leu |
| **Ex c)** | Ala | Ile | Ile | Ile | Leu | Arg | Ile | Tyr | Leu | Leu | Met | Val |
| **Ex d)** | Ala | Leu | Met | Leu | Leu | Arg | Ile | Tyr | Leu | Leu | Ile | Met |
| **Ex e)** | Ala | Leu | Met | Ile | Ile | Arg | Val | Tyr | Leu | Leu | Ile | Met |
| **Ex f)** | Ala | Leu | Met | Ile | Ile | Arg | Ile | Phe | Leu | Leu | Ile | Met |
| **Ex g)** | Ala | Leu | Met | Ile | Val | Arg | Ile | Phe | Leu | Leu | Ile | Phe |
| **Ex h)** | Val | Leu | Met | Ile | Leu | Arg | Ile | Phe | Leu | Leu | Ile | Met |
| **Ex i)** | Ala | Leu | Met | Ile | Leu | Arg | Ile | Phe | Leu | Leu | Ile | Met |
| **Ex j)** | Ala | Leu | Met | Ile | Leu | Arg | Ile | Tyr | Leu | Leu | Ala | Ala |
| **Ex k)** | Val | Leu | Met | Ile | Leu | Arg | Ile | Tyr | Leu | Leu | Val | Leu |
| **Ex l)** | Val | Leu | Met | Ile | Leu | Arg | Ile | Phe | Leu | Leu | Val | Leu |
| | | | | | | | | | | | | |
|---|---|---|---|---|---|---|---|---|---|---|---|---|
| (Ex = exemple) | | | | | | | | | | | | |
les exposants des J, Z, U, X et B représentant la position de ces acides aminés dans ladite séquence.

2. Peptide selon la revendication 1, dans lequel les acides aminés J sont choisis indépendamment les uns des autres parmi Ala, Arg, Asn, Asp, Cys, Gln, Glu, Gly, His, Ile, Leu, Lys, Met, Phe, Pro, Ser, Thr Trp, Tyr et Val de telle manière que au moins 50% d'entre eux sont des résidus polaires choisis parmi Arg, Asn, Asp, Gln, Glu, Gly, His, Lys, Pro, Ser et Thr.

3. Peptide constitué d'une séquence choisie parmi les séquences IDn°1 à IDn°10 de la liste de séquences annexée.

4. Peptide constitué de la séquence IDn°1 de la liste de séquence annexée.

5. Peptide selon l'une quelconque des revendications 1 à 4, comportant en outre, liée à l'extrémité N-terminale de la séquence, une séquence d'acides aminés choisie parmi Gly-Ser-Cys-, Gly-Ser-Thr-, Gly-Ser-Pro-, Gly-Ser-Ser-, Gly-Ser-Gly-, et Gly-Ser-Gln-.

6. Peptide selon l'une quelconque des revendications 1 à 4, comportant en outre, liée à l'extrémité N-terminale de la séquence, une séquence d'acides aminés Gly-Ser-Gly-Cys-, Gly-Cys-Gly-Ser, Gly-Ser-Gly-Ser, Gly-Cys-Gly-Cys, Gly-Cys-Gly-Ser.

7. Peptide constitué de la séquence IDn°11 ou IDn°12 de la liste de séquences annexée.

8. Peptide constitué de la séquence IDn°13 ou IDn°14 de la liste de séquences annexée.

9. Procédé de fabrication d'un peptide selon l'une quelconque des revendication 1 à 8, ledit procédé comprenant une synthèse chimique en phase solide dudit peptide

10. Procédé de fabrication en culture d'un peptide selon l'une des revendications 1 à 8, ledit procédé comprenant les étapes suivantes :
a) préparation d'un cDNA comprenant une séquence de base codant pour ledit peptide
b) insertion dudit cDNA dans un vecteur d'expression approprié,
c) transformation d'une cellule hôte appropriée, avec ledit vecteur dans lequel le cDNA a été inséré, pour une réplication du plasmide,
d) fabrication dudit peptide par traduction dudit cDNA dans ladite cellule hôte, et
e) récupération du peptide synthétisé.

11. Procédé selon la revendication 10, dans lequel le vecteur est un plasmide.

12. Procédé selon la revendication 10, dans lequel le vecteur est le vecteur pGEX-2T.

13. Procédé selon la revendication 10, 11 ou 12, dans lequel la cellule hôte est *E*. *Coli*.

14. Assemblage chimique ayant une affinité pour un phospholipide, **caractérisé en ce qu'**il comprend au moins deux peptides tels que définis dans les revendications 1 à 8, identiques ou différentes, lesdits peptides étant liés entre eux.

15. Assemblage chimique selon la revendication 14, dans lequel au moins un des peptides est un des peptides définis dans la revendication 3.

16. Utilisation d'un peptide selon l'une quelconque des revendications 1 à 8 pour le recouvrement d'un biomatériau.

17. Utilisation d'un peptide selon l'une quelconque des revendications 1 à 8 dans la fabrication d'un filtre pour le piégeage de cellules sanguines circulantes activées.

18. Composé de marquage comprenant un peptide tel que définie dans l'une quelconque des revendications 1 à 8 couplé à une molécule de marquage ou à des nanoparticules denses en microscopie électronique.

19. Composé de marquage **caractérisé en ce qu'**il comprend un assemblage tel que défini dans la revendication 14 ou 15 couplé à une molécule de marquage ou à des nanoparticules denses en microscopie électronique.

20. Composé selon la revendication 18 ou 19 dans lequel la molécule de marquage est une molécule fluorescente.

21. Composé selon la revendication 18 ou 19 dans lequel la molécule de marquage est constituée d'un des partenaires du système avidine-biotine.

22. Composé selon la revendication 18 ou 19 dans lequel la molécule de marquage est un radioélément.

23. Composé selon la revendication 18 ou 19 dans lequel la molécule de marquage est un agent de contraste en imagerie à résonance magnétique.

24. Composé selon la revendication 18 ou 19 dans lequel la molécule de marquage est du technétium.

25. Composé selon la revendication 18 ou 19 dans lequel les nanoparticules denses en microscopie électronique sont des nanoparticules d'or.

26. Trousse de diagnostic comprenant un composé selon l'une quelconque des revendications 18 ou 19.

27. Trousse de diagnostic selon la revendication 26, comprenant en outre un réactif adéquat permettant de détecter ladite molécule de marquage.

28. Trousse d'analyse et de détection de charges négatives à la surface de cellules, **caractérisée en ce qu'**elle comprend un peptide selon l'une quelconque des revendications 1 à 8.

29. Trousse d'analyse et de détection de charges négatives à la surface de cellules, **caractérisée en ce qu'**elle comprend un assemblage selon la revendication 14 ou 15.

30. Trousse d'analyse et de détection de microvésicules dans le sang, **caractérisée en ce qu'**elle comprend un peptide selon l'une quelconque des revendications 1 à 8.

31. Trousse d'analyse et de détection de microvésicules dans le sang, **caractérisée en ce qu'**elle comprend un assemblage selon la revendication 14 ou 15.

32. Trousse selon la revendication 28 ou 30, dans laquelle le peptide est couplé à un marqueur.

33. Trousse selon la revendication 29 ou 31, dans laquelle l'assemblage est couplé à un marqueur.

34. Filtre de dialyse de cellules sanguines circulantes activées, ledit filtre étant **caractérisé en ce qu'**il comprend un peptide selon l'une quelconque des revendications 1 à 8.

## Claims

1. Peptide **characterized in that** it consists of the peptide sequence below:
J¹-J²-J³-J⁴-J⁵-J⁶-Asp-U⁸-J⁹-J¹⁰-U¹¹-Arg-J¹³-Ala-U¹⁵-Lys-Gly-X¹⁸-Gly-Thr-J²¹-Glu-J²³-J²⁴-U²⁵-J²⁶-J²⁷-J²⁸-U²⁹-J³⁰-J³¹-Arg-J³³-J³⁴-J³⁵-J³⁶-B³⁷-Gln-J³⁹-U⁴⁰-J⁴¹-J⁴²-J⁴³-U⁴⁴-J⁴⁵-J⁴⁶-J⁴⁷-J⁴⁸-J⁴⁹-Arg-J⁵¹-U⁵²-J⁵³-J⁵⁴-Asp-U⁵⁶-Lys-Ser-Z⁵⁹-Leu-J⁶¹-Gly-J⁶³-J⁶⁴-Z⁶⁵-J⁶⁶-J⁶⁷-U⁶⁸-J⁶⁹-J⁷⁰-J⁷¹-U⁷²-J⁷³-J⁷⁴-Ser
in which J, Z, U, X and B represent amino acids such that:
- the amino acids J are chosen, independently of one another, from natural amino acids or derivatives thereof, such that at least 50% of them are polar residues chosen from Arg, Asn, Asp, Cys, Gln, Glu, Gly, His, Lys, Orn, Pro, Ser, Thr and Tyr,
- the amino acid X¹⁸ is chosen, independently of the other amino acids of the sequence, from Ala, Asn, Cys, Gln, Gly, His, Ile, Leu, Met, Phe, Ser, Thr, Trp, Tyr and Val,
- the amino acids Z⁵⁹ and Z⁶⁵ are chosen, independently, from Glu, Asp, Lys or Arg,
- the amino acids U and B of the sequence are chosen according to one of the examples a) to j) disclosed in table 1 below:
| | U⁸ | U¹¹ | U¹⁵ | U²⁵ | U²⁹ | B³⁷ | U⁴⁰ | U⁴⁴ | U⁵² | U⁵⁶ | U⁶⁸ | U⁷² |
|---|---|---|---|---|---|---|---|---|---|---|---|---|
| Ex a) | Val | Leu | Met | Ile | Leu | Arg | Ile | Tyr | Leu | Leu | Val | Leu |
| Ex b) | Ala | Ile | Ile | Ile | Leu | Arg | Ile | Tyr | Leu | Leu | Ile | Leu |
| Ex c) | Ala | Ile | Ile | Ile | Leu | Arg | Ile | Tyr | Leu | Leu | Met | Val |
| Ex d) | Ala | Leu | Met | Leu | Leu | Arg | Ile | Tyr | Leu | Leu | Ile | Met |
| Ex e) | Ala | Leu | Met | Ile | Ile | Arg | Val | Tyr | Leu | Leu | Ile | Met |
| Ex f) | Ala | Leu | Met | Ile | Ile | Arg | Ile | Phe | Leu | Leu | Ile | Met |
| Ex g) | Ala | Leu | Met | Ile | Val | Arg | Ile | Phe | Leu | Leu | Ile | Phe |
| Ex h) | Val | Leu | Met | Ile | Leu | Arg | Ile | Phe | Leu | Leu | Ile | Met |
| Ex i) | Ala | Leu | Met | Ile | Leu | Arg | Ile | Phe | Leu | Leu | Ile | Met |
| Ex j) | Ala | Leu | Met | Ile | Leu | Arg | Ile | Tyr | Leu | Leu | Ala | Ala |
| Ex k) | Val | Leu | Met | Ile | Leu | Arg | Ile | Tyr | Leu | Leu | Val | Leu |
| Ex l) | Val | Leu | Met | Ile | Leu | Arg | Ile | Phe | Leu | Leu | Val | Leu |
| | | | | | | | | | | | | |
|---|---|---|---|---|---|---|---|---|---|---|---|---|
| (Ex = example) | | | | | | | | | | | | |
the superscripts of J, Z, U, X and B representing the position of these amino acids in said sequence.

2. Peptide according to Claim 1, in which the amino acids J are chosen, independently of one another, from Ala, Arg, Asn, Asp, Cys, Gln, Glu, Gly, His, Ile, Leu, Lys, Met, Phe, Pro, Ser, Thr, Trp, Tyr and Val, such that at least 50% of them are polar residues chosen from Arg, Asn, Asp, Gln, Glu, Gly, His, Lys, Pro, Ser and Thr.

3. Peptide consisting of a sequence chosen from the sequences ID No. 1 to ID No. 10 of the sequence listing in the appendix.

4. Peptide consisting of the sequence ID No. 1 of the sequence listing in the appendix.

5. Peptide according to any one of Claims 1 to 4, also comprising, linked to the N-terminal end of the sequence, an amino acid sequence chosen from Gly-Ser-Cys-, Gly-Ser-Thr-, Gly-Ser-Pro-, Gly-Ser-Ser-, Gly-Ser-Gly-, and Gly-Ser-Gln-.

6. Peptide according to any one of Claims 1 to 4, also comprising, linked to the N-terminal end of the sequence, an amino acid sequence Gly-Ser-Gly-Cys-, Gly-Cys-Gly-Ser-, Gly-Ser-Gly-Ser-, Gly-Cys-Gly-Cys- or Gly-Cys-Gly-Ser-.

7. Peptide consisting of the sequence ID No. 11 or ID No. 12 of the sequence listing in the appendix.

8. Peptide consisting of the sequence ID No. 13 or ID No. 14 of the sequence listing in the appendix.

9. Process for producing a peptide according to any one of Claims 1 to 8, said process comprising solid-phase chemical synthesis of said peptide.

10. Process for producing a peptide according to one of Claims 1 to 8, in culture, said process comprising the following steps:
a) preparing a cDNA comprising a basic sequence encoding said peptide,
b) inserting said cDNA into a suitable expression vector,
c) transforming a suitable host cell with said vector into which the cDNA has been inserted, for replication of the plasmid,
d) producing said peptide by translation of said cDNA in said host cell, and
e) recovering the synthesized peptide.

11. Process according to Claim 10, in which the vector is a plasmid.

12. Process according to Claim 10, in which the vector is the vector pGEX-2T.

13. Process according to Claim 10, 11 or 12, in which the host cell is *E. coli.*

14. Chemical assembly with affinity for a phospholipid, **characterized in that** it comprises at least two peptides as defined in Claims 1 to 8, which may be identical or different, said peptides being linked to one another.

15. Chemical assembly according to Claim 14, in which at least one of the peptides is one of the peptides defined in Claim 3.

16. Use of a peptide according to any one of Claims 1 to 8, for covering a biomaterial.

17. Use of a peptide according to any one of Claims 1 to 8, in the production of a filter for trapping activated circulating blood cells.

18. Labelling compound comprising a peptide as defined in any one of Claims 1 to 8, coupled to a labelling molecule or to nanoparticles that are dense in electron microscopy.

19. Labelling compound **characterized in that** it comprises an assembly as defined in Claim 14 or 15, coupled to a labelling molecule or to nanoparticles that are dense in electron microscopy.

20. Compound according to Claim 18 or 19, in which the labelling molecule is a fluorescent molecule.

21. Compound according to Claim 18 or 19, in which the labelling molecule consists of one of the partners of the avidin-biotin system.

22. Compound according to Claim 18 or 19, in which the labelling molecule is a radio element.

23. Compound according to Claim 18 or 19, in which the labelling molecule is a contrast agent in magnetic resonance imaging.

24. Compound according to Claim 18 or 19, in which the labelling molecule is technetium.

25. Compound according to Claim 18 or 19, in which the nanoparticles that are dense in electron microscopy are gold nanoparticles.

26. Diagnostic kit comprising a compound according to either one of Claims 18 or 19.

27. Diagnostic kit according to Claim 26, also comprising a suitable reagent for detecting said labelling molecule.

28. Kit for analysing and detecting negative charges at the surface of cells, **characterized in that** it comprises a peptide according to any one of Claims 1 to 8.

29. Kit for analysing and detecting negative charges at the surface of cells, **characterized in that** it comprises an assembly according to Claim 14 or 15.

30. Kit for analysing and detecting microvesicules in the blood, **characterized in that** it comprises a peptide according to any one of Claims 1 to 8.

31. Kit for analysing and detecting microvesicules in the blood, **characterized in that** it comprises an assembly according to Claim 14 or 15.

32. Kit according to Claim 28 or 30, in which the peptide is coupled to a label.

33. Kit according to Claim 29 or 31, in which the assembly is coupled to a label.

34. Filter for dialysing activated circulating blood cells, said filter being **characterized in that** it comprises a peptide according to any one of claims 1 to 8.

## Patentansprüche

1. Peptid **dadurch gekennzeichnet, dass** es sich aus der folgenden Peptidsequenz zusammensetzt:
J¹-J²-J³-J⁴-J⁵-J⁶-Asp-U⁸-J⁹-J¹⁰-U¹¹-Arg-J¹³-Ala-U¹⁵-Lys-Gly-X¹⁸-Gly-Thr-J²¹-Glu-J²³-J²⁴-U²⁵-J²⁶-J²⁷-J²⁸-U²⁹-J³⁰-J³¹-Arg-J³³-J³⁴-J³⁵-J³⁶-B³⁷-Gln-J³⁹-U⁴⁰-J⁴¹-J⁴²-J⁴³-U⁴⁴-J⁴⁵-J⁴⁶-J⁴⁷-J⁴⁸-J⁴⁹-Arg-J⁵¹-U⁵²-J⁵³-J⁵⁴-Asp-U⁵⁶-Lys-Ser-Z⁵⁹-Leu-J⁶¹-Gly-J⁶³-J⁶⁴-Z⁶⁵-J⁶⁶-J⁶⁷-U⁶⁸-J⁶⁹-J⁷⁰-J⁷¹-U⁷²-J⁷³-J⁷⁴-Ser
worin: J, Z, U, X und B wie folgt Aminosäuren darstellen:
- die Aminosäuren J sind unabhängig voneinander aus den natürlichen Aminosäuren oder deren Derivaten auf eine solche Weise ausgewählt, dass mindestens 50 % davon aus Arg, Asn, Asp, Cys, Gln, Glu, Gly, His, Lys, Orn, Pro, Ser, Thr und Tyr ausgewählte polare Reste sind,
- die Aminosäure X¹⁸ ist unabhängig von den anderen Aminosäuren der Sequenz aus Ala, Asn, Cys, Gln, Gly, His, Ile, Leu, Met, Phe, Ser, Thr, Trp, Tyr und Val ausgewählt,
- die Aminosäuren Z⁵⁹ und Z⁶⁵ sind unabhängig aus Glu, Asp, Lys oder Arg ausgewählt,
- die Aminosäuren U und B der Sequenz sind gemäß einem der in der folgenden Tabelle 1 dargestellten Beispiele a) bis j) ausgewählt:
| | **U⁸** | **U¹¹** | **U¹⁵** | **U²⁵** | **U²⁹** | **B³⁷** | **U⁴⁰** | **U⁴⁴** | **U⁵²** | **U⁵⁶** | **U⁶⁸** | **U⁷²** |
|---|---|---|---|---|---|---|---|---|---|---|---|---|
| **Ex a)** | Val | Leu | Met | Ile | Leu | Arg | Ile | Tyr | Leu | Leu | Val | Leu |
| **Ex b)** | Ala | Ile | Ile | Ile | Leu | Arg | Ile | Tyr | Leu | Leu | Ile | Leu |
| **Ex c)** | Ala | Ile | Ile | Ile | Leu | Arg | Ile | Tyr | Leu | Leu | Met | Val |
| **Ex d)** | Ala | Leu | Met | Leu | Leu | Arg | Ile | Tyr | Leu | Leu | Ile | Met |
| **Ex e)** | Ala | Leu | Met | Ile | Ile | Arg | Val | Tyr | Leu | Leu | Ile | Met |
| **Ex f)** | Ala | Leu | Met | Ile | Ile | Arg | Ile | Phe | Leu | Leu | Ile | Met |
| **Ex g)** | Ala | Leu | Met | Ile | Val | Arg | Ile | Phe | Leu | Leu | Ile | Phe |
| **Ex h)** | Val | Leu | Met | Ile | Leu | Arg | Ile | Phe | Leu | Leu | Ile | Met |
| **Ex i)** | Ala | Leu | Met | Ile | Leu | Arg | Ile | Phe | Leu | Leu | Ile | Met |
| **Ex j)** | Ala | Leu | Met | Ile | Leu | Arg | Ile | Tyr | Leu | Leu | Ala | Ala |
| **Ex k)** | Val | Leu | Met | Ile | Leu | Arg | Ile | Tyr | Leu | Leu | Val | Leu |
| **Ex l)** | Val | Leu | Met | Ile | Leu | Arg | Ile | Phe | Leu | Leu | Val | Leu |
| | | | | | | | | | | | | |
|---|---|---|---|---|---|---|---|---|---|---|---|---|
| (Ex = Beispiel), | | | | | | | | | | | | |
wobei die Exponenten von J, Z, U, X und B die Position dieser Aminosäuren in der Sequenz bedeuten.

2. Peptid gemäß Anspruch 1, bei dem die Aminosäuren J unabhängig voneinander aus Ala, Arg, Asn, Asp, Cys, Gln, Glu, Gly, His, Ile, Leu, Lys, Met, Phe, Pro, Ser, Thr, Trp, Tyr und Val auf eine solche Weise ausgewählt, dass mindestens 50 % davon aus Arg, Asn, Asp, Gln, Glu, Gly, His, Lys, Pro, Ser und Thr ausgewählte polare Reste sind.

3. Peptid, das sich aus einer Sequenz zusammensetzt, die aus den Sequenzen ID Nr. 1 bis ID Nr. 10 der angefügten Sequenzliste ausgewählt ist.

4. Peptid, das sich aus der Sequenz ID Nr. 1 der angefügten Sequenzliste zusammensetzt.

5. Peptid gemäß einem der Ansprüche 1 bis 4, das außerdem eine an das N-terminale Ende der Sequenz gebundene Aminosäuresequenz umfasst, die aus Gly-Ser-Cys-, Gly-Ser-Thr-, Gly-Ser-Pro-, Gly-Ser-Ser-, Gly-Ser-Gly- und Gly-Ser-Gln- ausgewählt ist.

6. Peptid gemäß einem der Ansprüche 1 bis 4, das außerdem eine an das N-terminale Ende der Sequenz gebundene Aminosäuresequenz umfasst, die aus Gly-Ser-Gly-Cys-, Gly-Cys-Gly-Ser, Gly-Ser-Gly-Ser, Gly-Cys-Gly-Cys und Gly-Cys-Gly-Ser ausgewählt ist.

7. Peptid, das sich aus der Sequenz ID Nr. 11 oder ID Nr. 12 der angefügten Sequenzliste zusammensetzt.

8. Peptid, das sich aus der Sequenz ID Nr. 13 oder ID Nr. 14 der angefügten Sequenzliste zusammensetzt.

9. Verfahren zur Herstellung eines Peptids gemäß einem der Ansprüche 1 bis 8, wobei das Verfahren eine chemische Festphasensynthese umfasst.

10. Verfahren zur Herstellung in Kultur eines Peptids gemäß einem der Ansprüche 1 bis 8, wobei das Verfahren die folgenden Schritte umfasst:
a) Herstellung einer cDNA, die eine für das Peptid kodierende Basensequenz umfasst,
b) Insertion der cDNA in einen geeigneten Expressionsvektor,
c) Transformation einer geeigneten Wirtszelle mit dem Vektor, in den die cDNA inseriert ist, zur Replikation des Plasmids,
d) Herstellung des Peptids durch Transduktion der cDNA in der Wirtszelle und
e) Isolieren des synthetisierten Peptids.

11. Verfahren gemäß Anspruch 10, wobei der Vektor ein Plasmid ist.

12. Verfahren gemäß Anspruch 10, wobei der Vektor der Vektor pGEX-2T ist.

13. Verfahren gemäß Anspruch 10, 11 oder 12, wobei die Wirtszelle *E. coli* ist.

14. Chemischer Verbund mit einer Affinität für ein Phospholipid, **dadurch gekennzeichnet, dass** er wenigstens zwei wie in den Ansprüchen 1 bis 8 definierte, gleiche oder verschiedene Peptide umfasst, wobei die Peptide miteinander verbunden sind.

15. Chemischer Verbund gemäß Anspruch 14, bei dem wenigstens eines der Peptide eines der in Anspruch 3 definierten Peptide ist.

16. Verwendung eines Peptids gemäß einem der Ansprüche 1 bis 8 zum Überziehen eines Biomaterials.

17. Verwendung eines Peptids gemäß einem der Ansprüche 1 bis 8 bei der Herstellung eines Filters zum Sammeln aktivierter Blutkreislaufzellen.

18. Markierungsverbindung, die ein wie in einem der Ansprüche 1 bis 8 definiertes Peptid umfasst, das an ein Markierungsmolekül oder an elektronenmikroskopisch dichte Nanopartikel gekoppelt ist.

19. Markierungsverbindung, **dadurch gekennzeichnet, dass** sie einen wie in Anspruch 14 oder 15 definierten Verbund umfasst, der an ein Markierungsmolekül oder an elektronenmikroskopisch dichte Nanopartikel gekoppelt ist.

20. Verbindung gemäß Anspruch 18 oder 19, bei der das Markierungsmolekül ein fluoreszierendes Molekül ist.

21. Verbindung gemäß Anspruch 18 oder 19, bei der sich das Markierungsmolekül aus einem der Partner des Systems Avidin-Biotin zusammensetzt.

22. Verbindung gemäß Anspruch 18 oder 19, bei der das Markierungsmolekül ein Radioelement ist.

23. Verbindung gemäß Anspruch 18 oder 19, bei der das Markierungsmolekül ein Kontrastmittel bei der Magnetresonanzbildgebung ist.

24. Verbindung gemäß Anspruch 18 oder 19, bei der das Markierungsmolekül Technetium ist.

25. Verbindung gemäß Anspruch 18 oder 19, bei der die elektronenmikroskopisch dichten Nanopartikel Goldnanopartikel sind.

26. Diagnostischer Kit umfassend eine Verbindung gemäß einem der Ansprüche 18 und 19.

27. Diagnostischer Kit gemäß Anspruch 26, der außerdem ein geeignetes Reagenz umfasst, das den Nachweis des Markierungsmoleküls gestattet.

28. Kit zur Analyse und zum Nachweis negativer Ladungen auf der Oberfläche von Zellen, **dadurch gekennzeichnet, dass** er ein Peptid gemäß einem der Ansprüche 1 bis 8 umfasst.

29. Kit zur Analyse und zum Nachweis negativer Ladungen auf der Oberfläche von Zellen, **dadurch gekennzeichnet, dass** er einen Verbund gemäß Anspruch 14 oder 15 umfasst.

30. Kit zur Analyse und zum Nachweis von Mikrovesikeln im Blut, **dadurch gekennzeichnet, dass** er ein Peptid gemäß einem der Ansprüche 1 bis 8 umfasst.

31. Kit zur Analyse und zum Nachweis von Mikrovesikeln im Blut, **dadurch gekennzeichnet, dass** er einen Verbund gemäß Anspruch 14 oder 15 umfasst.

32. Kit gemäß Anspruch 28 oder 30, bei dem das Peptid an einen Marker gekuppelt ist.

33. Kit gemäß Anspruch 29 oder 31, bei dem der Verbund an einen Marker gekuppelt ist.

34. Dialysefilter für aktivierte Blutkreislaufzellen, wobei das Filter **dadurch gekennzeichnet ist, dass** es ein Peptid gemäß einem der Ansprüche 1 bis 8 umfasst.


### LISTE DE SEQUENCES

<110> COMMISSARIAT A L'ENERGIE ATOMIQUE
   UNIVERSITE PIERRE ET MARIE CURIE (PARIS VI)
<120> PEPTIDES AYANT UNE AFFINITE POUR UN PHOSPHOLIPIDE ET UTILISATIONS
<130> B14001.3EE
<140>
   <141>
<150> FR N°02 08202
   <151> 2002-07-01
<160> 14
<170> PatentIn Ver. 2.1
<210> 1
   <211> 75
   <212> PRT
   <213> Séquence artificielle
<220>
   <223> Description de la séquence artificielle: séquence dérivée d'une annexine humaine
<400> 1
   Gly Phe Asp Glu Arg Ala Asp Val Glu Thr Leu Arg Lys Ala Met Lys
    1               5                  10                  15
   Gly Leu Gly Thr Asp Glu Glu Ser Ile Leu Thr Leu Leu Thr Ser Arg
               20                  25                  30
   Ser Asn Ala Gln Arg Gln Glu Ile Ser Ala Ala Tyr Lys Thr Leu Phe
           35                  40                  45
   Gly Arg Asp Leu Leu Asp Asp Leu Lys Ser Glu Leu Thr Gly Lys Phe
       50                  55                  60
   Glu Lys Leu Val Val Ala Leu Leu Lys Pro Ser
   65                  70                  75
<210> 2
   <211> 75
   <212> PRT
   <213> Séquence artificielle
<220>
   <223> Description de la séquence artificielle: séquence dérivée d'une annexine humaine
<400> 2
   Asn Phe Asp Ala Glu Arg Asp Ala Leu Asn Ile Arg Lys Ala Ile Lys
    1               5                  10                  15
   Gly Met Gly Val Asp Glu Asp Thr Ile Val Asn Ile Leu Thr Asn Arg
               20                  25                  30
   Ser Asn Ala Gln Arg Gln Asp Ile Ala Phe Ala Tyr Gln Arg Arg Thr
           35                  40                  45
   Lys Arg Glu Leu Ala Ser Asp Leu Lys Ser Glu Leu Ser Gly His Leu
       50                  55                  60
   Glu Arg Val Ile Leu Gly Leu Leu Lys Thr Ser
   65                  70                  75
<210> 3
   <211> 75
   <212> PRT
   <213> Séquence artificielle
<220>
   <223> Description de la séquence artificielle: séquence dérivée d'une annexine humaine
<400> 3
   Asp Phe Ser Pro Ser Val Asp Ala Glu Ala Ile Arg Lys Ala Ile Lys
   1               5                   10                  15
   Gly Ile Gly Thr Asp Glu Asp Met Leu Ile Ser Ile Leu Thr Glu Arg
               20                  25                  30
   Ser Asn Ala Gln Arg Gln Leu Ile Val Lys Glu Tyr Gln Ala Ala Tyr
           35                  40                  45
   Gly Arg Glu Leu Lys Asp Asp Leu Lys Ser Glu Leu Ser Gly His Phe
       50                  55                  60
   Glu Arg Leu Met Val Ala Leu Val Thr Pro Ser
   65                  70                  75
<210> 4
   <211> 75
   <212> PRT
   <213> Séquence artificielle
<220>
   <223> Description de la séquence artificielle: séquence dérivée d'une annexine humaine
<400> 4
   Gly Phe Asn Ala Met Glu Asp Ala Gln Thr Leu Arg Lys Ala Met Lys
   1 5 10 15
   Gly Leu Gly Thr Asp Glu Asp Ala Ile Ile Ser Val Leu Ala Tyr Arg
   20 25 30
   Asn Thr Ala Gln Arg Gln Glu Ile Arg Thr Ala Tyr Lys Ser Thr Ile
   35 40 45
   Gly Arg Asp Leu Ile Asp Asp Leu Lys Ser Glu Leu Ser Gly Asn Phe
   50 55 60
   Glu Arg Val Ile Val Gly Met Met Thr Pro Ser
   65 70 75
<210> 5
   <211> 75
   <212> PRT
   <213> Séquence artificielle
<220>
   <223> Description de la séquence artificielle: séquence dérivée d'une annexine humaine
<400> 5
   Gly Phe Asp Pro Asn Gln Asp Ala Glu Ala Leu Arg Thr Ala Met Lys
   1 5 10 15
   Gly Phe Gly Ser Asp Glu Glu Ala Ile Leu Asp Ile Ile Thr Ser Arg
   20 25 30
   Ser Asn Arg Gln Arg Gln Glu Val Cys Gln Ser Tyr Lys Ser Leu Tyr
   35 40 45
   Gly Arg Asp Leu Ile Ala Asp Leu Lys Ser Glu Leu Thr Gly Lys Phe
   50 55 60
   Glu Arg Leu Ile Val Gly Leu Met Arg Pro Ser
   65 70 75
<210> 6
   <211> 75
   <212> PRT
   <213> Séquence artificielle
<220>
   <223> Description de la séquence artificielle: séquence dérivée d'une annexine humaine
<400> 6
   Gly Phe Asn Pro Asp Ala Asp Ala Lys Ala Leu Arg Lys Ala Met Lys
   1 5 10 15
   Gly Leu Gly Thr Asp Glu Asp Thr Ile Ile Asp Ile Ile Thr His Arg
   20 25 30
   Ser Asn Val Gln Arg Gln Gln Ile Arg Gln Thr Phe Lys Ser His Phe
   35 40 45
   Gly Arg Asp Leu Met Thr Asp Leu Lys Ser Glu Ile Ser Gly Asp Leu
   50 55 60
   Glu Arg Leu Ile Leu Gly Leu Met Met Pro Ser
   65 70 75
<210> 7
   <211> 75
   <212> PRT
   <213> Séquence artificielle
<220>
   <223> Description de la séquence artificielle: séquence dérivée d'une annexine humaine
<400> 7
   Pro Gly Asp Ala Ile Arg Asp Ala Glu Ile Leu Arg Lys Ala Met Lys
   1 5 10 15
   Gly Phe Gly Thr Asp Glu Gln Ala Ile Val Asp Val Val Ala Asn Arg
   20 25 30
   Ser Asn Asp Gln Arg Gln Lys Ile Lys Ala Ala Phe Lys Thr Ser Tyr
   35 40 45
   Gly Arg Asp Leu Ile Lys Asp Leu Lys Ser Glu Leu Ser Gly Asn Met
   50 55 60
   Glu Arg Leu Ile Leu Ala Leu Phe Met Pro Ser
   65 70 75
<210> 8
   <211> 75
   <212> PRT
   <213> Séquence artificielle
<220>
   <223> Description de la séquence artificielle: séquence dérivée d'une annexine humaine
<400> 8
   His Phe Asn Pro Asp Pro Asp Val Glu Thr Leu Arg Lys Ala Met Lys
   1 5 10 15
   Gly Ile Gly Thr Asn Glu Gln Ala Ile Ile Asp Val Leu Thr Lys Arg
   20 25 30
   Ser Asn Thr Gln Arg Gln Thr Ile Ala Lys Ser Phe Lys Ala Gln Phe
   35 40 45
   Gly Arg Asp Leu Thr Glu Asp Leu Lys Ser Glu Leu Ser Gly Lys Leu
   50 55 60
   Glu Arg Leu Ile Val Ala Leu Met Tyr Pro Ser
   65 70 75
<210> 9
   <211> 75
   <212> PRT
   <213> Séquence artificielle
<220>
   <223> Description de la séquence artificielle: séquence dérivée d'une annexine humaine
<400> 9
   Gly Phe Asp Pro Leu Arg Asp Ala Glu Val Leu Arg Lys Ala Met Lys
   1 5 10 15
   Gly Phe Gly Thr Asp Glu Gln Ala Ile Ile Asp Cys Leu Gly Ser Arg
   20 25 30
   Ser Asn Lys Gln Arg Gln Gln Ile Leu Leu Ser Phe Lys Thr Ala Tyr
   35 40 45
   Gly Arg Asp Leu Ile Lys Asp Leu Lys Ser Glu Leu Ser Gly Asn Phe
   50 55 60
   Glu Lys Thr Ile Leu Ala Leu Met Lys Thr Ser
   65 70 75
<210> 10
   <211> 75
   <212> PRT
   <213> Séquence artificielle
<220>
   <223> Description de la séquence artificielle: séquence dérivée d'une annexine humaine
<400> 10
   Gly Phe Asp Val Asp Arg Asp Ala Lys Lys Leu Arg Lys Ala Met Lys
   1 5 10 15
   Gly Met Gly Thr Asn Glu Ala Ala Ile Ile Glu Ile Leu Ser Gly Arg
   20 25 30
   Thr Ser Asp Glu Arg Gln Gln Ile Lys Gln Lys Tyr Lys Ala Thr Tyr
   35 40 45
   Gly Arg Glu Leu Glu Glu Asp Leu Lys Ser Glu Leu Ser Gly Asn Phe
   50 55 60
   Glu Lys Thr Ala Leu Ala Leu Leu Asp Arg Ser
   65 70 75
<210> 11
   <211> 79
   <212> PRT
   <213> Séquence artificielle
<220>
   <223> Description de la séquence artificielle: séquence dérivée d'une annexine humaine
<220>
   <223> Xaa en position 22 est Leu, Met ou Trp
<220>
   <223> Xaa en position 34 est Thr ou Lys
<220>
   <223> Xaa en position 45 est Ser ou Lys
<220>
   <223> Xaa en position 48 est Phe ou Tyr
<220>
   <223> Xaa en position 50 est Thr ou Glu
<220>
   <223> Xaa en position 63 est Glu ou Lys
<220>
   <223> Xaa en position 69 est Glu ou Lys
<220>
   <223> Xaa en position 71 est Glu ou Leu
<400> 11
   Gly Ser Gly Cys Gly Phe Asp Glu Arg Ala Asp Val Glu Thr Leu Arg
   1 5 10 15
   Lys Ala Met Lys Gly Xaa Gly Thr Asp Glu Glu Ser Ile Leu Thr Leu
   20 25 30
   Leu Xaa Ser Arg Ser Asn Ala Gln Arg Gln Glu Ile Xaa Ala Ala Xaa
   35 40 45
   Lys Xaa Leu Phe Gly Arg Asp Leu Leu Asp Asp Leu Lys Ser Xaa Leu
   50 55 60
   Thr Gly Lys Phe Xaa Lys Xaa Val Val Ala Leu Leu Lys Pro Ser
   65 70 75
<210> 12
   <211> 78
   <212> PRT
   <213> Séquence artificielle
<220>
   <223> Description de la séquence artificielle: séquence dérivée d'une annexine humaine
<400> 12
   Gly Ser Pro Gly Phe Asp Glu Arg Ala Asp Val Glu Thr Leu Arg Lys
   1 5 10 15
   Ala Met Lys Gly Leu Gly Thr Asp Glu Glu Ser Ile Leu Thr Leu Leu
   20 25 30
   Thr Ser Arg Ser Asn Ala Gln Arg Gln Glu Ile Ser Ala Ala Tyr Lys
   35 40 45
   Thr Leu Phe Gly Arg Asp Leu Leu Asp Asp Leu Lys Ser Glu Leu Thr
   50 55 60
   Gly Lys Phe Glu Lys Leu Val Val Ala Leu Leu Lys Pro Ser
   65 70 75
<210> 13
   <211> 83
   <212> PRT
   <213> Séquence artificielle
<220>
   <223> Description de la séquence artificielle: séquence dérivée d'une annexine humaine
<220>
   <223> Xaa en position 25 est Leu, Met ou Trp
<220>
   <223> Xaa en position 37 est Thr ou Lys
<220>
   <223> Xaa en position 48 est Ser ou Lys
<220>
   <223> Xaa en position 51 est Phe ou Tyr
<220>
   <223> Xaa en position 53 est Thr ou Glu
<220>
   <223> Xaa en position 66 est Glu ou Lys
<220>
   <223> Xaa en position 72 est Glu ou Lys
<220>
   <223> Xaa en position 74 est Glu ou Leu
<400> 13
   Gly Ser Glu Cys Asp Phe Pro Gly Phe Asp Glu Arg Ala Asp Val Glu
    1               5                  10                  15
   Thr Leu Arg Lys Ala Met Lys Gly Xaa Gly Thr Asp Glu Glu Ser Ile
               20                  25                  30
   Leu Thr Leu Leu Xaa Ser Arg Ser Asn Ala Gln Arg Gln Glu Ile Xaa
           35                  40                  45
   Ala Ala Xaa Lys Xaa Leu Phe Gly Arg Asp Leu Leu Asp Asp Leu Lys
       50                  55                  60
   Ser Xaa Leu Thr Gly Lys Phe Xaa Lys Xaa Val Val Ala Leu Leu Lys
   65                  70                  75                  80
   Pro Ser Arg
<210> 14
   <211> 87
   <212> PRT
   <213> Séquence artificielle
<220>
   <223> Description de la séquence artificielle: séquence dérivée d'une annexine humaine
<220>
   <223> Xaa en position 29 est Leu, Met ou Trp
<220>
   <223> Xaa en position 41 est Tyr ou Lys
<220>
   <223> Xaa en position 52 est Ser ou Lys
<220>
   <223> Xaa en position 55 est Phe ou Tyr
<220>
   <223> Xaa en position 57 est Thr ou Glu
<220>
   <223> Xaa en position 70 est Glu ou Lys
<220>
   <223> Xaa en position 76 est Glu ou Lys
<220>
   <223> Xaa en position 78 est Glu ou Leu
<400> 14
   Gly Ser Gly Cys Gly Thr Glu Thr Asp Phe Pro Gly Phe Asp Glu Arg
    1               5                  10                  15
   Ala Asp Val Glu Thr Leu Arg Lys Ala Met Lys Gly Xaa Gly Thr Asp
               20                  25                  30
   Glu Glu Ser Ile Leu Thr Leu Leu Xaa Ser Arg Ser Asn Ala Gln Arg
           35                  40                  45
   Gln Glu Ile Xaa Ala Ala Xaa Lys Xaa Leu Phe Gly Arg Asp Leu Leu
       50                  55                  60
   Asp Asp Leu Lys Ser Xaa Leu Thr Gly Lys Phe Xaa Lys Xaa Val Val
   65                  70                  75                  80
   Ala Leu Leu Lys Pro Ser Arg
                   85